# EUROPEAN PATENT APPLICATION

(11) **EP 4 238 556 A1**
(43) Date of publication of application: **06.09.2023**
(21) Application number: 21885314.1
(22) Date of filing: 29.10.2021
(51) Int. Cl.: A61K 9/22, A61K 9/52, A61K 9/48, A61K 9/70, A61D 7/00, A61J 3/07

(54) **GASTRORETENTIVE PHARMACEUTICAL DOSAGE FORM**

(30) Priority: 30.10.2020 WO PCT/CN2020/125319; 27.10.2021 CN 202111255791
(71) Applicant: Triastek, Inc., Nanjing, Jiangsu 211111 (CN)
(72) Inventor: DENG, Feihuang, Nanjing, Jiangsu 211111 (CN); ZHENG, Yu, Nanjing, Jiangsu 211111 (CN); YAO, Juan, Nanjing, Jiangsu 211111 (CN); LI, Xiaoling, Dublin, California 94568 (US); CHENG, Senping, Nanjing, Jiangsu 211111 (CN); ZUO, Xianghao, Nanjing, Jiangsu 211111 (CN)
(74) Representative: Lahrtz, Fritz
(86) International application number: PCT/CN2021/127458
(87) International publication number: WO 2022/089588

(57) **Abstract**

A pharmaceutical dosage form, comprising: a pharmaceutical unit which includes a medicament, at least one unfolding member, and a retaining member, wherein the unfolding member is connected to the pharmaceutical unit, and is constructed to have a contracted shape, which makes the unfolding member become closer to the pharmaceutical unit, and an unfolding shape, which makes the unfolding member become farther away from the pharmaceutical unit; the retaining member is connected to the at least one unfolding member, and applies a constraint force to the at least one unfolding member, so as to make the unfolding member be in the contracted shape; and the retaining member contains a water-soluble material, and is constructed to remove the constraint force from the unfolding member when the water-soluble material is dissolved, such that the at least one unfolding member can change from the contracted shape to the unfolding shape.

## Description

### TECHNICAL FIELD

The present application relates to pharmaceutical dosage forms, and particularly to a pharmaceutical dosage form capable of achieving retention in the stomach or other parts of the digestive tract.

### BACKGROUND

The retention time of the drug at the part to be acted in the human body is one of the important factors affecting the final treatment result. However, rapid gastric emptying and short gastrointestinal running time often result in the traditional oral drug delivery system failing to achieve effective retention at the part to be acted, thus affecting the final bioavailability of the drug. In some cases, the drug is expelled from the stomach or upper gastrointestinal tract even before being completely released, severely affecting the therapeutic effect. In order to solve the above problems, drug delivery systems capable of retaining in the stomach and other parts have been developed, which mainly rely on increasing the structural size, improving the surface adhesion or changing the overall density to achieve the retention of the drug in the gastrointestinal tract, thereby decreasing dosing frequency, improving drug efficacy, and further increasing patient compliance.

However, the existing drug delivery systems capable of being retained in the gastrointestinal tract generally have complicated dosage forms and high manufacturing costs. Meanwhile, the specific application process of some existing retentive drug delivery systems also needs to be assisted by structures such as gastroesophageal tubes, stomach tubes or capsules, which require specialized drug administration skills or drug loading procedures, compromising ease of use and productivity.

### SUMMARY

The present application provides a pharmaceutical dosage form capable of achieving retention in the stomach or other parts of the digestive tract, thereby prolonging the retention time of the drug in the part to be acted, decreasing dosing frequency, increasing patient tolerance and improving the clinical efficacy.

In one aspect of the present application, provided is a pharmaceutical dosage form including: a pharmaceutical unit containing a drug; at least one unfolding member connected to the pharmaceutical unit and configured to have a contracted shape proximal to the pharmaceutical unit and an unfolding shape distal to the pharmaceutical unit; and a retaining member connected to the at least one unfolding member and applying a constraint force to the at least one unfolding member to enable the unfolding member to be in the contracted shape; wherein the retaining member contains a water-soluble material, and the retaining member is configured to remove the constraint force from the unfolding member when the water-soluble material is dissolved, such that the at least one unfolding member can change from the contracted shape to the unfolding shape.

In some embodiments, the unfolding member includes at least one elastic portion configured such that the unfolding member is in the contracted shape proximal to the pharmaceutical unit under the action of the constraint force, and returns to the unfolding shape distal to the pharmaceutical unit when the constraint force is withdrawn.

In some embodiments, the retaining member at least partially encases the pharmaceutical unit and the at least one unfolding member, such that the at least one unfolding member is in the contracted shape.

In some embodiments, the retaining member is disposed between the pharmaceutical unit and the unfolding member and is connected to the pharmaceutical unit, such that the unfolding member is in the contracted shape.

In some embodiments, the pharmaceutical dosage form includes a plurality of unfolding members, wherein the retaining member is connected to at least two unfolding members, such that the at least two unfolding members are in the contracted shape.

In some embodiments, the elastic portion is located at one end of the unfolding member adjacent to the pharmaceutical unit.

In some embodiments, the unfolding member has a portion with reduced thickness at the end adjacent to the pharmaceutical unit.

In some embodiments, the unfolding member has a strip, sheet, curved, petal, or rod shape.

In some embodiments, the pharmaceutical dosage form includes a plurality of unfolding members substantially uniformly distributed along the periphery of the pharmaceutical unit.

In some embodiments, the pharmaceutical unit includes a hollow chamber connected to the pharmaceutical unit. In some embodiments, the hollow chamber is connected to the unfolding member. In some embodiments, the hollow chamber is contained in the pharmaceutical unit.

In some embodiments, the pharmaceutical unit includes an outer shell and a drug-containing inner core.

In some embodiments, the outer shell of the pharmaceutical unit contains the same host material as the unfolding member.

In another aspect of the present application, provided is a method for preparing a pharmaceutical dosage form using a three-dimensional printing process, which includes: printing a pharmaceutical unit containing a drug and at least one unfolding member, such that the unfolding member is connected to the pharmaceutical unit; configuring the unfolding member to have a contracted shape proximal to the pharmaceutical unit from an unfolding shape; and directly printing a retaining member, such that the retaining member is connected to at least one unfolding member and applies the constraint force to the at least one unfolding member to enable the unfolding member to be in the contracted shape; wherein the retaining member contains a water-soluble material, and the retaining member is configured to remove the constraint force from the unfolding member when the water-soluble material is dissolved, such that the at least one unfolding member can change from the contracted shape to the unfolding shape.

In some embodiments, the at least one unfolding member is printed to include at least one elastic portion configured such that the unfolding member is in the contracted shape proximal to the pharmaceutical unit under the action of the constraint force, and returns to the unfolding shape distal to the pharmaceutical unit when the constraint force is withdrawn.

In some embodiments, the printing the retaining member includes: printing the retaining member, such that the retaining member at least partially encases or bonds with the pharmaceutical unit and the at least one unfolding member, such that the at least one unfolding member is in the contracted shape.

In some embodiments, the printing the retaining member includes: printing the retaining member, such that the retaining member is disposed between the pharmaceutical unit and the unfolding member and is connected to the pharmaceutical unit, such that the unfolding member is in the contracted shape.

In some embodiments, the steps of printing the at least one unfolding member and printing the retaining member are performed simultaneously or alternately by different print heads.

In some embodiments, the unfolding member is printed to have a portion with reduced thickness at one end adjacent to the pharmaceutical unit.

In some embodiments, the printing the at least one unfolding member includes: printing the at least one unfolding member in the unfolding shape; and moving the at least one unfolding member, such that the unfolding member is in the contracted shape.

In some embodiments, the pharmaceutical unit includes a first portion containing a drug and a second portion containing no drug, wherein the first portion containing the drug is printed after the step of printing the retaining member is completed.

In another aspect of the present application, provided is a pharmaceutical dosage form comprising: a pharmaceutical unit comprising a drug, wherein at least a portion of the pharmaceutical unit has a bent or folded shape; a retaining member connected to the pharmaceutical unit and applying a constraint force to the pharmaceutical unit to enable at least a portion of the pharmaceutical unit to retain the bent or folded shape; wherein the retaining member contains a water-soluble material, and the retaining member is configured to remove the constraint force from the pharmaceutical unit when the water-soluble material is dissolved, thereby allowing at least a portion of the pharmaceutical unit to unfold from the bent or folded shape.

In some embodiments, the pharmaceutical unit is configured to have a helical shape with a radius of curvature gradually increasing from the inside to the outside, and the retaining member at least partially fills a gap of the helical shape of the pharmaceutical unit.

In some embodiments, the pharmaceutical unit includes an outer shell layer and a drug layer, wherein the outer shell layer is composed of a hydrophobic material.

In some embodiments, the drug layer contains a hydrophilic material.

In some embodiments, the drug layer includes a first portion and a second portion, wherein the first portion and the second portion contain different drugs.

In some embodiments, the pharmaceutical unit is generally in an elongated shape when unfolded.

In yet another aspect of the present application, provided is a method for preparing a pharmaceutical dosage form using a three-dimensional printing process, which includes: printing a pharmaceutical unit containing a drug, such that at least a portion of the pharmaceutical unit has a bent or folded shape; and printing a retaining member, such that the retaining member is connected to the pharmaceutical unit and applies a constraint force to the pharmaceutical unit to enable at least a portion of the pharmaceutical unit to retain the bent or folded shape; wherein the retaining member contains a water-soluble material, and the retaining member is configured to remove the constraint force from the pharmaceutical unit when the water-soluble material is dissolved, thereby allowing at least a portion of the pharmaceutical unit to unfold from the bent or folded shape.

In some embodiments, the pharmaceutical unit is configured to have a helical shape with a radius of curvature gradually increasing from the inside to the outside, and the retaining member at least partially fills a gap of the helical shape of the pharmaceutical unit.

In some embodiments, the pharmaceutical unit includes an outer shell layer and a drug layer, wherein the outer shell layer is composed of a hydrophobic material.

In one aspect of the present application, provided is a pharmaceutical dosage form comprising: an unfolding member comprising at least one elastic portion configured such that the unfolding member is in a contracted shape under the action of a constraint force, and returns to an unfolding shape when the constraint force is withdrawn; and a retaining member surrounded the unfolding member and applying the constraint force to the unfolding member to enable the unfolding member to retain the contracted shape; wherein the retaining member contains a water-soluble material, and the retaining member is configured to remove the constraint force from the unfolding member when the water-soluble material is dissolved, thereby allowing the unfolding member to at least partially unfold.

In some embodiments, the retaining member includes a first portion and a second portion, the unfolding member is connected to the first portion, the second portion contains a water-soluble material, and the retaining member removes the constraint force from the unfolding member when the water-soluble material of the second portion is dissolved, thereby allowing the unfolding member to at least partially unfold.

In some embodiments, the first portion contains a first drug and the second portion contains a second drug.

In some embodiments, the first portion contains at least one compartment, and the first drug is disposed in the compartment.

In some embodiments, the unfolding member includes a first portion and a second portion, and the first portion of the unfolding member has a dissolution rate in gastric juice less than the second portion of the retaining member in gastric juice.

In some embodiments, the first portion of the unfolding member contains a drug.

In some embodiments, the unfolding member further includes a third portion, wherein the first portion of the unfolding member is connected to the second portion of the unfolding member by the third portion of the unfolding member, and the first portion of the unfolding member is disconnected from the second portion when the third portion is dissolved.

In some embodiments, the second portion and the third portion of the unfolding member contain a hydrophobic material and an enteric material.

In some embodiments, the first portion of the unfolding member substantially extends along a first direction, the second portion of the unfolding member substantially extends along a second direction, and the first portion and the second portion of the unfolding member are cross-connected to each other.

In some embodiments, the first portion of the unfolding member includes at least one internal compartment, wherein a drug is disposed in the internal compartment.

In some embodiments, the elastic portion of the unfolding member is configured to substantially extend along a plane when the unfolding member is in a contracted configuration.

In some embodiments, the pharmaceutical dosage form has a length greater than or equal to 16 mm at least along one direction when the water-soluble material of the retaining member is at least partially dissolved.

In some embodiments, the unfolding member is configured to fully unfold within at least 10 min after entering gastric juice.

In one aspect of the present application, provided is a method for preparing a pharmaceutical dosage form using a three-dimensional printing process, which includes: printing an unfolding member including at least one elastic portion configured such that the unfolding member is in a contracted shape under the action of a constraint force, and returns to an unfolding shape when the constraint force is withdrawn; applying the constraint force to the unfolding member to enable the unfolding member to retain the contracted shape; and printing a retaining member to retain the constraint force on the unfolding member by the retaining member to enable the unfolding member to retain the contracted shape; wherein the retaining member contains a water-soluble material, and the retaining member is configured to remove the constraint force from the unfolding member when the water-soluble material is dissolved, thereby allowing the unfolding member to at least partially unfold.

In some embodiments, the retaining member includes a first portion and a second portion, and the method further includes: printing a first portion of the retaining member after printing the unfolding member, such that the unfolding member is connected to the first portion of the retaining member; and applying the constraint force to the unfolding member by the first portion of the retaining member to enable the unfolding member to retain the contracted shape.

In some embodiments, the method further includes: printing a second portion of the retaining member, wherein the second portion contains a water-soluble material, and the retaining member removes the constraint force from the unfolding member when the water-soluble material of the second portion is dissolved, thereby allowing the unfolding member to at least partially unfold.

In one aspect of the present application, provided is a method for preparing a pharmaceutical dosage form using a three-dimensional printing process, which includes: printing a retaining member; and printing an unfolding member under the action of a constraint force of the retaining member within a range surrounded by the retaining member, such that the unfolding member is connected to the retaining member, wherein the unfolding member includes at least one elastic portion configured to enable the unfolding member to at least partially unfold when the constraint force applied by the retaining member is removed; wherein the retaining member contains a water-soluble material, and the retaining member is configured to remove the constraint force from the unfolding member when the water-soluble material is dissolved, thereby allowing the unfolding member to at least partially unfold.

In some embodiments, the printed unfolding member is subjected to a heat treatment to increase the elasticity of the elastic portion.

The foregoing is a summary of the present application that may be subject to simplification, generalization, and omission of details, and thus it should be understood by those skilled in the art that this section is illustrative only and is not intended to limit the scope of the present application in any way. This summary is not intended to identify key features or essential features of the claimed subject matter, nor is it intended to be used as an aid in determining the scope of the claimed subject matter.

### BRIEF DESCRIPTION OF THE DRAWINGS

The above and other features of the present disclosure will be more fully and clearly understood from the following description and appended claims, in combination with the drawings. It can be understood that these drawings depict only several embodiments of the present disclosure and therefore should not be considered to limit the scope of the contents of the present application. The present disclosure will be described more clearly and in detail by using the drawings.
FIG. 1 shows a schematic perspective view of a pharmaceutical dosage form 100 according to one embodiment of the present application with its unfolding members 101 in an unfolding shape;
FIG. 2 shows a schematic perspective view of the pharmaceutical dosage form 100 shown in FIG. 1 with its unfolding members 101 in a contracted shape;
FIG. 3 shows a schematic perspective view of a pharmaceutical dosage form 200 according to one embodiment of the present application with its unfolding member 201 not in a fully contracted shape;
FIG. 4 shows a schematic perspective view of the pharmaceutical dosage form 200 shown in FIG. 3 with its unfolding member 201 in a contracted shape;
FIG. 5 shows a schematic perspective view of a pharmaceutical dosage form 300 according to one embodiment of the present application with its unfolding members 301 in an unfolding shape;
FIG. 6 shows a schematic perspective view of the pharmaceutical dosage form 300 shown in FIG. 5 with its unfolding members 301 in a contracted shape;
FIG. 7 shows a schematic perspective view of a pharmaceutical dosage form 400 according to one embodiment of the present application with its unfolding member 401 in an unfolding shape;
FIG. 8 shows a schematic perspective view of the pharmaceutical dosage form 400 shown in FIG. 7 with its unfolding member 401 in a contracted shape;
FIG. 9 shows a schematic top view of a pharmaceutical dosage form 500 according to an embodiment of the present application;
FIG. 10 shows a schematic top view of a pharmaceutical dosage form 600 according to another embodiment of the present application;
FIG. 11 shows a schematic top view of a pharmaceutical dosage form 700 according to one embodiment of the present application with an unfolding member 701 in an unfolding shape after a retaining member is removed;
FIG. 12 shows a schematic top view of the pharmaceutical dosage form 700 shown in FIG. 11;
FIG. 13 shows a schematic top view of a pharmaceutical dosage form 800 according to one embodiment of the present application in an unfolding shape after a retaining member is removed;
FIG. 14 shows a schematic front view of the pharmaceutical dosage form 800 shown in FIG. 13 in an unfolding shape after a retaining member is removed;
FIG. 15 shows a schematic front view of the pharmaceutical dosage form 800 shown in FIG. 13;
FIG. 16 shows a schematic cross-sectional top view of the pharmaceutical dosage form 800 shown in FIG. 13;
FIG. 17 shows a schematic perspective view of a pharmaceutical dosage form 900 according to one embodiment of the present application in an unfolding shape after a retaining member is removed;
FIG. 18 shows a schematic perspective view of the pharmaceutical dosage form 900 shown in FIG. 17 in a contracted shape after a retaining member is removed;
FIG. 19 shows a schematic perspective view of the pharmaceutical dosage form 900 shown in FIG. 17;
FIG. 20 shows a schematic cross-sectional front view of the pharmaceutical dosage form 900 shown in FIG. 17;
FIG. 21 shows a schematic cross-sectional front view of a pharmaceutical dosage form 1000 according to one embodiment of the present application;
FIG. 22 shows a schematic perspective view of a pharmaceutical dosage form 1100 according to one embodiment of the present application in an unfolding shape after a retaining member is removed;
FIG. 23 shows a schematic perspective view of the pharmaceutical dosage form 1100 shown in FIG. 22 in a contracted shape after a retaining member is removed;
FIG. 24 shows a schematic top view of the pharmaceutical dosage form 1100 shown in FIG. 22;
FIG. 25 shows a schematic cross-sectional front view of the pharmaceutical dosage form 1100 shown in FIG. 22;
FIG. 26 shows a schematic cross-sectional front view of a pharmaceutical dosage form 1200 according to one embodiment of the present application;
FIG. 27 shows a schematic top view of a pharmaceutical dosage form 1300 according to one embodiment of the present application;
FIG. 28 shows a schematic cross-sectional front view of the pharmaceutical dosage form 1300 shown in FIG. 27.
FIG. 29 shows a schematic cross-sectional front view of a pharmaceutical dosage form 1400 according to one embodiment of the present application;
FIG. 30 shows a schematic top view of a pharmaceutical dosage form 1500 according to one embodiment of the present application;
FIG. 31 shows a schematic perspective view of the pharmaceutical dosage form 1500 shown in FIG. 30 unfolded in a strip shape after a retaining member is removed;
FIG. 32 shows a schematic top view of a pharmaceutical dosage form 1600 according to one embodiment of the present application;
FIG. 33 shows a schematic perspective view of the pharmaceutical dosage form 1600 shown in FIG. 32 unfolded in a strip shape after a retaining member is removed;
FIG. 34 shows a schematic top view of a pharmaceutical dosage form 1700 according to an embodiment of the present application;
FIG. 35 shows a schematic top view of the pharmaceutical dosage form 1700 shown in FIG. 34 with an unfolding member 1703 in an unfolding shape after a retaining member is removed;
FIG. 36 shows schematic diagrams of structures of three pharmaceutical dosage forms similar to the pharmaceutical dosage form shown in FIG. 34;
FIG. 37 shows a photograph of the pharmaceutical dosage form 1700 shown in FIG. 34 in an actual unfolding state;
FIG. 38 shows a schematic top view of a pharmaceutical dosage form 1800 according to an embodiment of the present application;
FIG. 39 shows a schematic top view of the pharmaceutical dosage form 1800 shown in FIG. 38 with an unfolding member in an unfolding shape after a portion of a retaining member is removed;
FIG. 40 shows a schematic top view of a pharmaceutical dosage form 1900 according to an embodiment of the present application;
FIG. 41 shows a schematic top view of the pharmaceutical dosage form 1900 shown in FIG. 40 with an unfolding member in an unfolding shape after a retaining member is removed;
FIG. 42 shows a 3D modeling of the pharmaceutical dosage form 1900 shown in FIG. 40;
FIG. 43 shows pictures of an unconstrained unfolding member, a pharmaceutical dosage form (c), and an unfolding pharmaceutical dosage form of the pharmaceutical dosage form 1900 shown in FIG. 40;
FIG. 44 shows a size-time response curve corresponding to the pharmaceutical dosage form 1900 shown in FIG. 40;
FIG. 45 shows size-time response curves corresponding to the pharmaceutical dosage form having different elastic portion configurations shown in FIG. 36.

### DETAILED DESCRIPTION

In the following detailed description, reference is made to the drawings, which form a part hereof. The illustrative embodiments described in the detailed description, drawings, and claims are not intended to be limiting. Other embodiments may be used, and other changes may be made, without departing from the spirit or scope of the subject matter of the present application. Before the present application is described in greater detail, it should be understood that the present application is not limited to particular embodiments described, as such may, of course, vary. It should also be understood that the terms used herein are for the purpose of describing particular embodiments only, and are not intended to be limiting, since the scope of the present application will be limited only by the appended claims.

Where a range of values is provided, it is understood that each intervening value between the upper and lower limit of that range and any other stated or intervening value in that stated range, to the tenth of the unit of the lower limit, unless the context clearly dictates otherwise, is encompassed within the application. The upper and lower limits of these smaller ranges may independently be included in the smaller ranges, and are also included in the range of the present application, subject to any specifically excluded limit in the stated range. Where the stated range includes one or both of the limits, ranges excluding either or both of those included limits are also included in the present application. Reference herein to an "about" value or parameter includes (and describes) variations that are directed to that value or parameter itself. For example, a description referring to "about X" includes a description of "X".

All publications and patents cited in the present specification are incorporated herein by reference as if each individual publication or patent is specifically and individually indicated to be incorporated by reference and is set forth in its entirety herein to disclose and describe the methods and/or materials in connection with which the publications were cited. Any publications cited are disclosed herein before the filing date of the present application, and the dates of publication provided may be different from the actual dates of publication, which may need to be individually identified.

As will be understood by those of skill in the art upon reading the present application, various embodiments described and illustrated herein have discrete compositions and characteristics which may be readily separated from or combined with the characteristics of any of the other several embodiments without departing from the scope or spirit of the present application. Any described method may be performed in the order of events recited or in any other order that is logically possible.

Before the embodiments of the present application are described in detail, it should be understood that unless otherwise explicitly indicated, the present application is not limited to particular materials, reagents, reaction starting materials, production processes, etc., which can vary. It should also be understood that the terms used herein are for the purpose of describing particular embodiments only, and are not intended to be limiting. It is also possible in the present application to perform the steps in a logically possible different order.

The term "comprising", "including", "containing", and "having" and other similar expressions used herein shall be equivalent in meaning, and the subsequent listing of one or more elements after these terms is not intended to be exhaustive or limited to the one or more elements listed. For example, an article "including" components A, B, and C can consist of (i.e., contain only) components A, B, and C, or can contain not only components A, B, and C, but also one or more other components.

It should be noted that, as used in this specification and the appended claims, the singular forms "a", "an", and "the" include plural referents, unless otherwise clearly indicated in the context. Thus, for example, "a compound" includes a plurality of compounds. In this specification and in the claims below, reference will be made to a plurality of terms that shall be defined to have the following meanings unless the contrary is clearly intended.

### Pharmaceutical Dosage Form I

The present application provides a pharmaceutical dosage form capable of achieving retention in the stomach or other parts of the digestive tract, thereby prolonging the retention time of the drug in the part to be acted, decreasing dosing frequency, increasing patient tolerance and improving the clinical efficacy. In some embodiments, the pharmaceutical dosage form may be formed by printing with a 3D printing method.

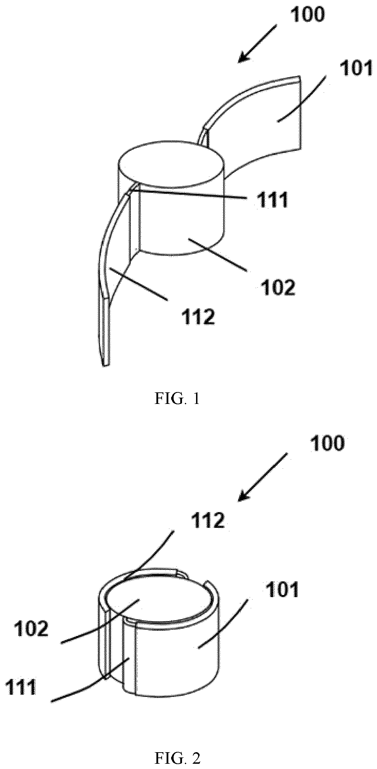

FIG. 1 shows a schematic perspective view of a pharmaceutical dosage form 100 according to one embodiment of the present application with its unfolding members 101 in an unfolding shape. FIG. 2 shows a schematic perspective view of the pharmaceutical dosage form 100 shown in FIG. 1 with its unfolding members 101 in a contracted shape.

As shown in FIGs. 1 and 2, the pharmaceutical dosage form 100 includes a pharmaceutical unit 102 and two unfolding members 101 connected thereto. The unfolding member 101 has an elastic portion 111 and an inelastic portion 112. The elastic portion 111 is configured such that the unfolding member 101 is in a contracted shape proximal to the pharmaceutical unit 102, as shown in FIG. 2, under the action of a constraint force, and returns to an unfolding shape distal to the pharmaceutical unit 102, as shown in FIG. 1, when the constraint force is withdrawn.

Although not shown in FIG. 2, in some embodiments, the pharmaceutical dosage form 100 further includes a retaining member connected to the unfolding member 101. The retaining member applies a constraint force to the unfolding member 101 to enable it to be in the contracted shape as shown in FIG. 2. The retaining member comprises a water-soluble material and is configured such that when the water-soluble material is dissolved in an aqueous solution or body fluid, the retaining member may be at least partially detached or collapsed, thereby removing the constraint force from the unfolding member 101. This enables the unfolding member 101 to move to an unfolding shape as shown in FIG. 1.

In use, the pharmaceutical dosage form 100, constrained by the retaining member to a contracted shape as shown in FIG. 2, is delivered orally or by other means of administration to a part to be applied in the human body (e.g., the stomach or other parts of the upper digestive tract). Subsequently, due to the rapid dissolution of the water-soluble material in the retaining member, the unfolding members 101 move to the unfolding shape as shown in FIG. 1, resulting in an increase in the overall structural size thereof. Since the structural size of the unfolding member in the unfolding shape is generally less likely to pass through a relatively narrow segment of the part to be applied (e.g., the pylorus of the stomach), retention of the pharmaceutical dosage form 100 at the part to be applied is achieved, which prolongs its retention time and improves bioavailability.

The unfolding size of the pharmaceutical dosage form 100 is set to be suitable for providing the retention effect described above. Specifically, when used to provide a retention effect, the pharmaceutical dosage form has an unfolding size of greater than or equal to 12 mm, preferably greater than or equal to 18 mm. The "unfolding size" described herein refers to the maximum linear distance between any two points on the pharmaceutical dosage form 100 in the unfolding shape.

It should be noted that although the pharmaceutical dosage form 100 as shown in FIGs. 1 and 2 includes the elastic portions 111 to facilitate the return of the unfolding members 101 from the contracted shape as shown in FIG. 2 to the unfolding shape as shown in FIG. 1 when the constraint force is withdrawn, in some embodiments, the pharmaceutical dosage form 100 may not include the elastic portion 111, and is configured to require an external force to move from the contracted shape to the unfolding shape. Specifically, in some embodiments, an elastic material is not used for the elastic portions 111 as shown in FIGs. 1 and 2 of the pharmaceutical dosage form 100, and instead, the thickness of the portion is set to be less than the thickness of the adjacent portion. Since the portion with reduced thickness is relatively easy to bend, the unfolding members 101 can still easily unfold from the contracted shape as shown in FIG. 2 to the unfolding shape as shown in FIG. 1 under the action of gastric juice or other substances. By using the pharmaceutical dosage form 100 constructed with the portion with reduced thickness, a similar unfolding effect can be achieved without particularly employing an elastic material, thereby effectively reducing costs. In addition, since there is no need to employ an elastic material, when the pharmaceutical dosage form is printed by using a 3D printing technique, its unfolding member can be printed with the same material as a whole, avoiding multiple switching of the printing head. Meanwhile, the pharmaceutical dosage form can also be printed directly in a contracted shape, which effectively improves the production efficiency of the final product.

### Unfolding Member

The unfolding members 101 as shown in FIGs. 1 and 2 are in a strip shape, and move from an unfolding shape to a contracted shape by wrapping around the cylindrical outer peripheral wall of the pharmaceutical unit 102. In some embodiments, the unfolding members 101 may also have a petal, rod, plate, or spiral shape, or any other shape suitable for bending or folding. In some embodiments, the unfolding members 101 may move from the unfolding shape to the contracted shape by bending or folding up and down, zigzag folding, winding in any direction, and the like.

Although two unfolding members 101 are included in the illustrated pharmaceutical dosage form 100, in some embodiments, the pharmaceutical dosage form may include any other number of unfolding members 101, e.g., 1, 2, 3, 4, 6, 7, 8, 9, and the like. The two unfolding members 101 as shown in FIG. 1 are substantially uniformly distributed around the peripheral wall of the pharmaceutical unit 102. In some embodiments, the pharmaceutical dosage form 101 may include 4 strip-shaped unfolding members substantially uniformly distributed along the periphery of the pharmaceutical unit, which move from an unfolding shape to a contracted shape by wrapping around the cylindrical periphery of the pharmaceutical unit 102.

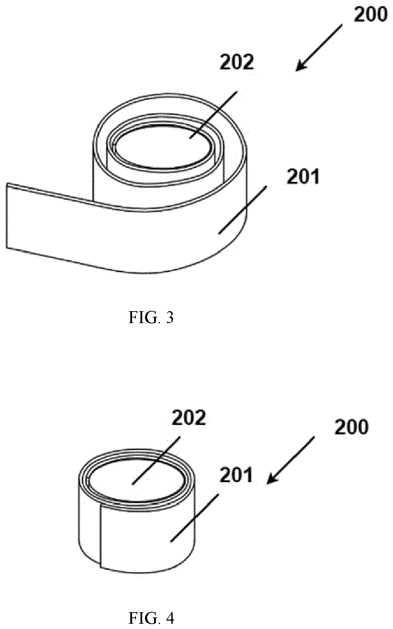

FIG. 3 shows a schematic perspective view of a pharmaceutical dosage form 200 according to one embodiment of the present application with its unfolding member 201 not in a fully contracted shape. FIG. 4 shows a schematic perspective view of the pharmaceutical dosage form 200 shown in FIG. 3 with its unfolding member 201 in a contracted shape.

Specifically, in the embodiment as shown in FIGs. 3 and 4, the pharmaceutical dosage form 200 has a relatively long strip-shaped unfolding member 201, and the strip-shaped unfolding member 201 is wound around the periphery of a pharmaceutical unit 202 in one direction to move from an unfolding shape to a contracted shape as shown in FIG. 4.

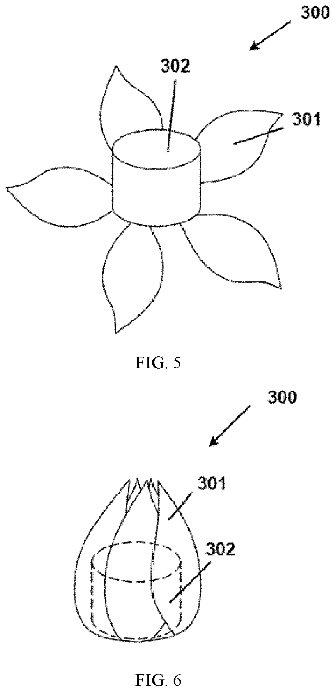

FIG. 5 shows a schematic perspective view of a pharmaceutical dosage form 300 according to one embodiment of the present application with its unfolding members 301 in an unfolding shape. FIG. 6 shows a schematic perspective view of the pharmaceutical dosage form 300 shown in FIG. 5 with its unfolding members 301 in a contracted shape.

Specifically, in the embodiment as shown in FIGs. 5 and 6, the pharmaceutical dosage form 300 has five petal-shaped unfolding members 301, and each petal-shaped unfolding member 301 has one end attached to the lower surface of a pharmaceutical unit 302 (or to the lower half part of the outer peripheral wall of the pharmaceutical unit 302) and the other end foldable toward the pharmaceutical unit 302. The upper ends of the plurality of petal-shaped unfolding members 301 are gathered together, such that the unfolding members move from the unfolding shape as shown in FIG. 5 to the contracted shape as shown in FIG. 6.

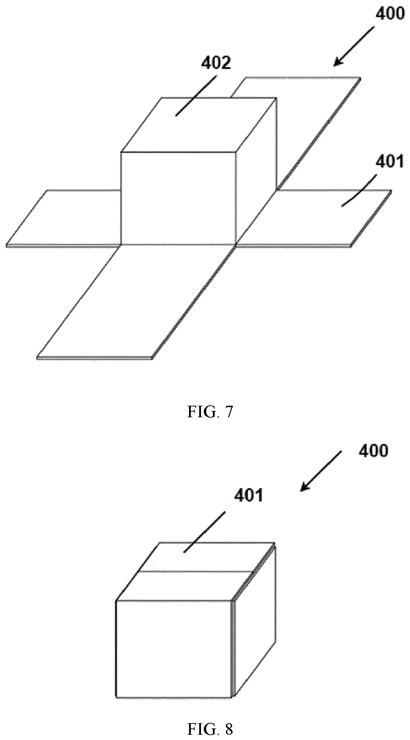

FIG. 7 shows a schematic perspective view of a pharmaceutical dosage form 400 according to one embodiment of the present application with its unfolding members 401 in an unfolding shape. FIG. 8 shows a schematic perspective view of the pharmaceutical dosage form 400 shown in FIG. 7 with its unfolding members 401 in a contracted shape.

Specifically, in the embodiment as shown in FIGs. 7 and 8, the pharmaceutical dosage form 400 has four strip-shaped unfolding members 401. Each unfolding member 401 has one end attached to one edge of the bottom of a pharmaceutical unit 402 which is in a cube or cuboid shape, and the other end of the strip-shaped unfolding member 401 is foldable toward the pharmaceutical unit 402, such that the unfolding member moves from the unfolding shape as shown in FIG. 7 to the contracted shape as shown in FIG. 8.

Although not shown in the figure, in some embodiments, the pharmaceutical dosage form may further include a floating member configured with one or more cavities; in other embodiments, the floating member may be configured in a honeycomb or grid shape having a filling rate of less than 100%, and the filling rate may be 10%, 20%, 30%, 40%, 50%, 60%, 70%, 80%, 90%, or the like, to provide buoyancy to the pharmaceutical dosage form in gastric juice, thereby further improving the retention effect of the pharmaceutical dosage form. In some embodiments, the floating member is connected to the pharmaceutical unit. In some embodiments, the floating member is connected to the unfolding mechanism. In some embodiments, the floating member is located inside the pharmaceutical unit. In some embodiments, the surface of the unfolding mechanism is a hydrophobic material. In some embodiments, the unfolding member may also contain a drug. In some embodiments, the unfolding member is printed by a 3D printing technique.

Referring to FIGs. 1 and 2 again, the elastic portions 111 of the unfolding members 101 are located at one end of the unfolding members 101 adjacent to the pharmaceutical unit 102, thereby facilitating pivoting of the unfolding members 101 about the elastic portions 111 between the unfolding shape and the contracted shape and enabling the unfolding members 101 as a whole to have a large pivot angle, such as 60 to 180 degrees, or preferably 90 to 120 degrees. In some other embodiments, the elastic portions may also be located elsewhere on the unfolding members 101, such as in a middle portion of the unfolding members 101 or at the other end distal to the pharmaceutical unit 102. In addition, in some embodiments, each unfolding member 101 may include a plurality of elastic portions spaced from one another, and each elastic portion enables the inelastic portion of the unfolding member 101 to which it is attached to deflect in the same or different directions. In some other embodiments, the unfolding member 101 as a whole may be composed of an elastic material to enable the unfolding member to contract to any desired contracted shape and more easily return to the unfolding shape automatically after the water-soluble material of the retaining member is dissolved. It will be understood that the unfolding shape may be achieved by adjusting the stress distribution or density of the elastic material and other manners.

Although the thickness of the elastic portions 111 of the unfolding members 101 as shown in FIGs. 1 and 2 is smaller than the thickness of the inelastic portions 112, in some embodiments, the elastic portions 111 may also have the same thickness as the inelastic portions 112. It should be noted that in some embodiments, the elastic portions 111 of the unfolding members 101 may be made of a different material to the inelastic portions 112, and the elastic portions 111 may contain an elastic material with a higher elastic coefficient. However, in some other embodiments, the elastic portions 111 may be made of the same material as the inelastic portions 112, and in the elastic portions, the unfolding members 101 may have a portion with reduced thickness, which is capable of providing the elastic deformation required to constrain the unfolding members 101 and the support force required to move the unfolding members 101 when releasing the unfolding members 101. In other words, since the portion with reduced thickness is relatively easy to bend, the unfolding members 101 still easily unfolds from the contracted shape as shown in FIG. 2 to the unfolding shape as shown in FIG. 1 under the action of gastric juice or other substances.

In some embodiments, the material used for the elastic portions 111 of the unfolding members 101 may be any elastic material suitable for 3D printing. Specifically, the elastic material may have a lower glass transition temperature (Tg), and generally has better elasticity, lower elasticity modulus, greater maximum elongation, higher toughness, or lower rigidity at normal temperature, thereby providing the properties of both plastics and rubber. The elastic portions 111 comprising the elastic material may be changed to any other shape by an external force and may be maximally drawn toward their original shape when the external force is removed. In addition, the elastic material can be processed into a melt at a high temperature and has less pronounced creep properties, so it is suitable for printing the elastic portion of the unfolding member in any desired shape or structure by a 3D printing method.

Specifically, the material used for the elastic portions 111 includes a polymer elastic material, which may be any one or a combination of materials listed below: shape memory polymer (SMP), liquid crystal elastomer (LCE), hydrogel (e.g., TecophilicTM from Lubrizol), thermoplastic elastomer (TPE) (e.g., styrenic block copolymers, TPS (TPE-s)), thermoplastic polyolefin elastomers (TPO (TPE-o)), thermoplastic vulcanizates (TPV (TPE-v or TPV)), thermoplastic polyurethanes (TPU (TPU)), thermoplastic copolyester (TPC (TPE-E)), thermoplastic polyamides (TPA (TPE-A)), polylactic acid (PLA), acrylonitrile-butadiene-styrene terpolymer (ABS), polyethylene (PE), polypropylene (PP), high impact polystyrene (HIPS), polyhexanedioic acid/butylene terephthalate (PBAT), acrylonitrile-butadiene-acrylate (ASA), polyamide (PA), polybutylene (PB), polyethylene terephthalate (PET), and polyvinylidene fluoride (PVDF).

TPE polymers mainly include CAWITON, THERMOLAST K, THERMOLAST M, Arnitel, Hytrel, Dryflex, Mediprene, Kraton, Pibiflex, Sofprene, and Laprene. Meanwhile, the styrenic block copolymers include CAWITON, THERMOLAST K, THERMOLAST M, Sofprene, Dryflex, and aprene. Thermoplastic polyurethanes include Laripur, Desmopan, and Elastollan. Thermoplastic vulcanizates include Sarlink, Santoprene, Termoton, Solprene, THERMOLAST V, Vegaprene, and Forprene. Thermoplastic olefin elastomers include For-Tec E, Engage, and Ninjaflex.

In addition to the above elastic materials, in some embodiments, the material used for the elastic portions 111 further contains a plasticizer. The plasticizer may be any one or a combination of materials listed below: triethyl citrate (TEC), vitamin E polyethylene glycol succinate (TPGS), acetin, acetylated triethyl citrate, tributyl citrate, tributyl o-acetyl citrate, polyhydroxy 15 hydroxystearate, polyoxyethylene-40 hydrogenated castor oil, polyoxyethylene-35 castor oil, dibutyl sebacate, diethyl phthalate, glycerol, methyl 4-hydroxybenzoate, castor oil, oleic acid, triacetin, and polyalkylene glycol.

In addition, the material used for the elastic portions 111 may further contain other additives, which may be any one or a combination of materials listed below: acacia, alginate, alginic acid, aluminum acetate, butyl paraben, butylated hydroxytoluene, citric acid, calcium carbonate, candelilla wax, croscarmellose sodium, fructose, colloidal silicon dioxide, cellulose, ordinary or anhydrous calcium phosphate, carnauba wax, corn starch, carboxymethylcellulose calcium, calcium disodium ethylenediaminetetraacetate (EDTA), dicalcium hydrogen phosphate dehydrate, cetylpyridine, dicalcium hydrogen phosphate, tricalcium phosphate, dicalcium hydrogen phosphate, disodium hydrogen phosphate, dimethicone, sodium erythrosine, ethylenediaminetetraacetic acid (EDTA), gelatin, monoolein, iron oxide, ferric oxide, iron oxide yellow, iron oxide red, lactose (aqueous, anhydrous, monohydrate, or spray-dried), microcrystalline cellulose, magnesium carbonate, magnesium oxide, methyl paraben, polysorbate 80, propyl paraben, potassium bicarbonate, potassium sorbate, potato starch, phosphoric acid, polyoxyethylene (40) stearate, sodium hydroxyacetate ferulate, pregelatinized starch, croscamellose sodium, sodium dodecyl sulfate, starch, silicon dioxide, sodium benzoate, sucrose, sorbic acid, sodium carbonate, sodium saccharin, sodium alginate, silica gel, sorbitan monooleate, sodium chloride, sodium metabisulfite, sodium citrate dehydrate, sodium starch, sodium carboxymethylcellulose, succinic acid, sodium propionate, titanium dioxide, and talc.

In some embodiments, the elastic portions 111 may be eroded, such that the pharmaceutical dosage form may be expelled from the stomach. In some embodiments, the elastic portions are completely eroded in the stomach with 8-72 h. In some embodiments, the elastic portions are completely eroded within at least 8 h, 10 h, 12 h, 14 h, 16 h, 18 h, 20 h, 22 h, 24 h, 36 h, 48 h, or 72 h in the stomach.

### Pharmaceutical Unit

The pharmaceutical unit 102 shown in FIGs. 1 and 2 is configured as a cylinder, but in some embodiments, the pharmaceutical unit 102 may also be in any other shape, including but not limited to, a capsule, a cylinder, a sphere, an ellipsoid, a triangular prism, a pentagonal prism, a bullet shape, an arrow shape, a tetrahedron, a cone, a truncated cone, an almond, a cuboid, a cube, or a combination thereof. In some embodiments, the size and shape of the pharmaceutical unit are configured to be suitable for oral administration to an individual.

In some embodiments, the pharmaceutical unit comprises an outer shell and a drug-containing inner core, and further comprises a drug-free plug or an intermediate layer; the pharmaceutical unit is made of a thermoplastic material, including a hydrophilic polymer, a hydrophobic polymer, a swellable polymer, a non-swelling polymer, a porous polymer, a nonporous polymer, an erodible polymer, or a non-erodible polymer.

In some embodiments, the pharmaceutical dosage form disclosed herein comprises at least one compartment in the pharmaceutical unit. The "compartment" as used herein refers to a space, a portion or a chamber defined or separated by the pharmaceutical unit. One compartment may be either sealed or open (i.e., with a hole). One compartment may be in any geometric shape that facilitates loading a drug. In some embodiments, the compartment may be in the shape of a pie, a pyramid, a column, or a cone. In some embodiments, the specially designed shape of the compartment enables the compartment to be capable of releasing the drug at a specific rate. In some embodiments, the compartment may be in the shape of a capsule, a wedge, a triangular prism, a pie, a pyramid, a column, a cube, an ellipse, or a cone.

In some embodiments, the pharmaceutical unit described herein comprises an unsealed compartment formed by an outer shell, and a drug-containing inner core is inside the compartment and comprises a drug and an extended-release material; when administered to a human body, the drug is released slowly. In some embodiments, the drug is completely released for at least 8 h, 10 h, 12h, 14 h, 16 h, 18 h, 20 h, 22 h, 24 h, 36 h, 48 h, or 72 h.

In some embodiments, the pharmaceutical unit described herein comprises an unclosed compartment formed by an outer shell material, a drug-containing inner core is inside the compartment, and the compartment is closed by a drug-free plug; when the drug is administered to a human body, the plug is first in contact with gastric juice and eroded, and the drug-containing inner core is exposed after the plug is eroded, so that a delayed release of the drug is achieved. In some embodiments, the drug-containing inner core has a multi-layer structure comprising a plurality of drug-containing layers and intermediate layers stacked to achieve a pulsed release of the drug.

In some embodiments, the pharmaceutical unit described herein comprises a plurality of compartments formed by an outer shell material, which are unclosed and placed side by side or in back-to-back contact. In some embodiments, the drug-containing inner core is partially surrounded by the outer shell. In some embodiments, the drug-containing inner core is separated by a portion of the shell or the intermediate layer. In some embodiments, the different compartments are unsealed by drug-free plugs. In some embodiments, the plugs are the same in length; in some embodiments, the plugs are different in length; in some embodiments, the materials of the plugs are the same; in some embodiments, the materials of the plugs are different. In some embodiments, the compartment does not comprise a plug.

### Drug-Containing Inner Core

In some embodiments, the drug-containing inner core comprises a drug and an extended-release material. In some embodiments, the drug-containing inner core is formed by mixing the drug and the extended-release material, hot-melt extrusion, and printing.

In some embodiments, the drug-containing inner core has a multi-layer structure comprising a drug-containing layer and an intermediate layer, which are alternately stacked. In some embodiments, the drug-containing layer is an immediate-release (IR) layer comprising the drug and having an immediate-release profile; in some embodiments, the drug-containing layer is an extended-release (ER) layer comprising the drug and having an extended-release profile. In some embodiments, the intermediate layer does not contain a drug and is used to separate the drug-containing layers to avoid cross-contamination of the drug or achieve a pulsed-release effect of the drug. In some embodiments, the drugs in each drug layer are the same. In some embodiments, the drugs in each drug layer are different. In some embodiments, the drug layer material is disposed in a first printing head, and the intermediate layer material is disposed in a second printing head, the first printing head and the second printing head printing the drug-containing inner core alternately layer by layer.

In some embodiments, the pharmaceutical unit comprises a plurality of compartments, and a first drug-containing inner core and a second drug-containing inner core are disposed in a first compartment and a second compartment, respectively; the first compartment and the second compartment are placed side by side or in back-to-back contact.

In some embodiments, the material mixed with the drug in the drug-containing inner core is a thermoplastic material. In some embodiments, the thermoplastic material is an erodible material, which may be selected from any one or a combination of the materials listed below:

copovidone (polyvinylpyrrolidone-vinyl acetate copolymer (PVP-VA) and polyvinylpyrrolidone-polyvinyl acetate copolymer (PVP-VA) 60/40), crospovidone, polyvinylpyrrolidone (PVP), hydroxypropylcellulose (HPC), hydroxypropyl methylcellulose or hypromellose (HPMC), hydroxypropyl methylcellulose phthalate (HPMCP), methylcellulose (MC), methacrylate copolymer, poly[butyl methacrylate, (2-dimethylaminoethyl)methyl methacrylate, methyl methacrylate] in a ratio of 1:2:1 (e.g., EUDRAGIT^{®}E 100, EUDRAGIT^{®}E 12,5, and EUDRAGIT^{®}E PO), poly[ethyl acrylate, methyl methacrylate] in a ratio of 2:1 (e.g., EUDRAGIT^{®}NE 30 D, EUDRAGIT^{®}NE 40 D, and EUDRAGIT^{®}NM 30 D), poly [methacrylic acid, methyl methacrylate] in a ratio of 1:1 (e.g., EUDRAGIT^{®}L 100, EUDRAGIT ^{®}L 12,5, and EUDRAGIT^{®}L 12,5 P), poly [methacrylic acid, ethyl acrylate] in a ratio of 1:1 (e.g., Acryl-EZE, Acryl-EZE 93A, Acryl-EZE MP, EUDRAGIT^{®}L 30 D -55, EUDRAGIT^{®}L 100-55, Eastacryl^{®}30 D, Kollicoat^{®}MAE 30 DP, and Kollicoat^{®}MAE 100 P), poly [methacrylic acid, methyl methacrylate] in a ratio of 1:2 (e.g., EUDRAGIT^{®}S 100, EUDRAGIT ^{®}S 12,5, and EUDRAGIT^{®}12,5 P), poly[methyl acrylate, methyl methacrylate, methacrylic acid] in a ratio of 7:3:1 (e.g., EUDRAGIT^{®}FS 30 D), poly[ethyl acrylate, methyl methacrylate, chlorotrimethylammoniumethyl methacrylate] in a ratio of 1:2:0.2 (e.g., EUDRAGIT^{®}RL 100, EUDRAGIT^{®} RL PO, EUDRAGIT^{®}RL 30 D, and EUDRAGIT^{®}RL 12,5), poly[ethyl acrylate, methyl methacrylate, chlorotrimethylammoniumethyl methacrylate] in a ratio of 1:2:0.1 (e.g. EUDRAGIT^{®}RS 100, EUDRAGIT^{®}RS PO, EUDRAGIT^{®}RS 30 D, and EUDRAGIT^{®}RS 12,5), polyethylene oxide (PEO), polyethylene glycol (PEG), polyvinyl caprolactam-polyvinyl acetate-polyethylene glycol graft copolymer 57/30/13, polyethylene glycol-polyvinyl alcohol graft copolymer 25/75 (Kollicoat^{®} IR), Kollicoat^{®} IR-polyvinyl alcohol 60/40 (Kollicoat^{®} Protect), polyvinyl alcohol (PVA), hydroxypropyl methylcellulose succinate or hypromellose acetate succinate (HPMCAS), ethylcellulose (EC), polyvinyl acetate (PVAc) and polyvinylpyrrolidone (PVP) 80/20, polyvinylacetal diethylamino lactate (AEA), carboxymethylethylcellulose (CMCE), carbomer, phospholipid, and cyclodextrin.

In some embodiments, the material mixed with the drug in the drug-containing inner core may further comprise a plasticizer and other additives, wherein the plasticizer may be selected from any one or a combination of the materials listed below: triethyl citrate (TEC), vitamin E polyethylene glycol succinate (TPGS), acetin, acetylated triethyl citrate, tributyl citrate, acetylated tributyl citrate, polyhydroxy 15 hydroxystearate, polyoxyethylene-40 hydrogenated castor oil, polyoxyethylene-35 castor oil, dibutyl sebacate, diethyl phthalate, glycerol, methyl 4-hydroxybenzoate, castor oil, oleic acid, triacetin, polyalkylene glycol, stearic acid, palmitic acid, and glyceryl monostearate. The other additives may be any one or a combination of the materials listed below: acacia, alginate, alginic acid, aluminum acetate, butyl paraben, butylated hydroxytoluene, citric acid, calcium carbonate, maydelilla wax, croscarmellose sodium, fructose, colloidal silicon dioxide, cellulose, ordinary or anhydrous calcium phosphate, carnauba wax, corn starch, carboxymethylcellulose calcium, calcium disodium ethylenediaminetetraacetate (EDTA), dicalcium hydrogen phosphate dehydrate, cetylpyridine, dicalcium hydrogen phosphate, tricalcium phosphate, dicalcium hydrogen phosphate, disodium hydrogen phosphate, dimethicone, sodium erythrosine, ethylenediaminetetraacetic acid (EDTA), gelatin, glyceryl oleate, iron oxide, iron sesquioxide, iron oxide yellow, iron oxide red, lactose (aqueous, anhydrous, monohydrate, or spray-dried), microcrystalline cellulose, magnesium carbonate, magnesium oxide, methyl paraben, polysorbate 80, propyl paraben, potassium bicarbonate, potassium sorbate, potato starch, phosphoric acid, polyoxyethylene (40) stearate, sodium arc glycolic acid, pregelatinized starch, croscamellose sodium, sodium dodecyl sulfate, starch, silicon dioxide, sodium benzoate, sucrose, sorbic acid, sodium carbonate, sodium saccharin, sodium alginate, silica gel, sorbitan monooleate, sodium chloride, sodium metabisulfite, sodium citrate dehydrate, sodium starch, sodium carboxymethylcellulose, succinic acid, sodium propionate, titanium dioxide, talc, magnesium stearate, and calcium stearate.

### Plug or Intermediate Layer

In some embodiments, the pharmaceutical unit described herein further comprises one or more plugs or intermediate layers for achieving a delayed release or a pulsed release of the drug. In some embodiments, the plug or intermediate layer is not mixed with the drug. In some embodiments, the plug or intermediate layer is in direct contact with the drug-containing layer. In some embodiments, the plug or intermediate layer is in direct contact with the shell. In some embodiments, the plug is in direct contact with the outer shell and the drug-containing layer. In some embodiments, the intermediate layer is in direct contact with the outer shell and the drug-containing layer. In some embodiments, the plug is located on the drug-containing layer such that when the drug is administered to a human body, the plug is first in contact with gastric juice and eroded. In some embodiments, the intermediate layer is located among the plurality of drug-containing layers.

In some embodiments, the plug or intermediate layer allows a delayed release or a pulsed release of the drug from the pharmaceutical unit. In some embodiments, the time for complete erosion of the plug or intermediate layer is about 1 min to about 12 h, such as any one of about 1 min to 10 min, about 10 min to about 30 min, about 30 min to about 1 h, about 1 h to about 3 h, about 3 h to about 6 h, about 6 h to about 9 h, and about 9 h to about 12 h. In some embodiments, the intermediate layer may reduce or avoid interference among two or more drug-containing layers contacting the intermediate layer.

In some embodiments, the dissolution rate (or erosion rate) of the intermediate layer is about 0.1 mm/h to about 50 mm/h. In some embodiments, the dissolution rate (or erosion rate) of the intermediate layer is at least about 0.1 mm/h, such as any one of at least about 1 mm/h, 5 mm/h, 10 mm/h, 20 mm/h, 30 mm/h, and 40 mm/h. In some embodiments, the dissolution rate (or erosion rate) of the intermediate layer is less than about 50 mm/h, such as any one of less than about 40 mm/h, 30 mm/h, 20 mm/h, 10 mm/h, 5 mm/h, and 1 mm/h.

In some embodiments, the intermediate layer is stacked on the drug-containing layer, wherein the intermediate layer has an upper surface and a lower surface, wherein the drug-containing layer has an upper surface and a lower surface, and wherein the shell is in direct contact with both the intermediate layer and the drug-containing layer. In some embodiments, the drug-containing layer is stacked on the intermediate layer. In some embodiments, the shell exposes the upper surface of the intermediate layer.

In some embodiments, the plug or intermediate layer has a slower dissolution rate than the drug-containing layer. In some embodiments, the plug or intermediate layer has a faster dissolution rate than the drug-containing layer. In some embodiments, the plug or intermediate layer has the same dissolution rate as the drug-containing layer.

In some embodiments, the plug or intermediate layer is composed of a thermoplastic material. In some embodiments, the thermoplastic material for the plug or intermediate layer is an erodible material. In some embodiments, the erodible material is selected from one or a combination of: polyvinyl caprolactam-polyvinyl acetate-polyethylene glycol graft copolymer 57/30/13, vinylpyrrolidone-vinyl acetate copolymer (PVP-VA), vinylpyrrolidone-vinyl acetate copolymer (PVP-VA) 60/40, polyvinylpyrrolidone (PVP), polyvinyl acetate (PVAc) and polyvinylpyrrolidone (PVP) 80/20, vinylpyrrolidone-vinyl acetate copolymer (VA64), polyethylene glycol-polyvinyl alcohol graft copolymer 25/75, kollicoat IR-polyvinyl alcohol 60/40, polyvinyl alcohol (PVA or PV-OH), poly(vinyl acetate) (PVAc), poly[butyl methacrylate, (2-dimethylaminoethyl)methyl methacrylate, methyl methacrylate] in a ratio of 1:2:1 (e.g., EUDRAGIT^{®}E 100, EUDRAGIT^{®}E 12,5, and EUDRAGIT^{®}E PO), poly[ethyl acrylate, methyl methacrylate] in a ratio of 2:1 (e.g., EUDRAGIT^{®}NE 30 D, EUDRAGIT^{®}NE 40 D, and EUDRAGIT^{®}NM 30 D), poly [methacrylic acid, methyl methacrylate] in a ratio of 1:1 (e.g., EUDRAGIT^{®}L 100, EUDRAGIT ^{®}L 12,5, and EUDRAGIT^{®}L 12,5 P), poly [methacrylic acid, ethyl acrylate] in a ratio of 1:1 (e.g., Acryl-EZE, Acryl-EZE 93A, Acryl-EZE MP, EUDRAGIT^{®}L 30 D -55, EUDRAGIT^{®}L 100-55, Eastacryl^{®}30 D, Kollicoat^{®}MAE 30 DP, and Kollicoat^{®}MAE 100 P), poly[methacrylic acid, methyl methacrylate] in a ratio of 1:2 (e.g., EUDRAGIT^{®}S 100, EUDRAGIT ^{®}S 12,5, and EUDRAGIT^{®}12,5 P), poly[methyl acrylate, methyl methacrylate, methacrylic acid] in a ratio of 7:3:1 (e.g., EUDRAGIT^{®}FS 30 D), poly[ethyl acrylate, methyl methacrylate, chlorotrimethylammoniumethyl methacrylate] in a ratio of 1:2:0.2 (e.g., EUDRAGIT^{®}RL 100, EUDRAGIT^{®} RL PO, EUDRAGIT^{®}RL 30 D, and EUDRAGIT^{®}RL 12,5), poly[ethyl acrylate, methyl methacrylate, chlorotrimethylammoniumethyl methacrylate] in a ratio of 1:2:0.1 (e.g. EUDRAGIT^{®}RS 100, EUDRAGIT^{®}RS PO, EUDRAGIT^{®}RS 30 D, and EUDRAGIT^{®}RS 12,5), polyethylene oxide (PEO), polyethylene glycol (PEG), hyperbranched polyesteramide, hydroxypropyl methylcellulose phthalate, hypromellose phthalate, hydroxypropyl methylcellulose or hypromellose (HMPC), hydroxypropyl methylcellulose acetate succinate or hypromellose succinate (HPMCAS), lactide-glycolide copolymer (PLGA), carbomer, ethylene-vinyl acetate copolymer, cellulose or cellulose derivatives, hydroxypropylcellulose (HPC), polyoxyethylene-40 hydrogenated castor oil, methylcellulose (MC), ethylcellulose (EC), poloxamer, hydroxypropyl methylcellulose phthalate (HPMCP), poloxamer, hydrogenated castor oil and soybean oil, glyceryl palmitostearate, carnauba wax, polyglycolic acid (PGA), cellulose acetate butyrate (CAB), colloidal silicon dioxide, saccharides, glucose, polyvinyl acetate phthalate (PVAP), wax, beeswax, hydrogel, gelatin, hydrogenated vegetable oil, polyvinylacetal diethylamino acetate (AEA), paraffin, shellac, sodium alginate, cellulose acetate phthalate (CAP), fatty oil, gum arabic, and xanthan gum.

In some embodiments, the thermoplastic material is mixed with a plasticizer or other additives. In some embodiments, the plasticizer or other additives is selected from one or a combination of: triethyl citrate (TEC), vitamin E polyethylene glycol succinate (TPGS), acetin, acetylated triethyl citrate, tributyl citrate, acetylated tributyl citrate, polyhydroxy 15 hydroxystearate, polyoxyethylene-40 hydrogenated castor oil, polyoxyethylene-35 castor oil, polyoxyethylene-polyoxypropylene block copolymer, dibutyl sebacate, diethyl phthalate, glycerol, methyl 4-hydroxybenzoate, castor oil, oleic acid, triacetin, polyalkylene glycol, hydroxystearate, polyethylene glycol (e.g., PEG400), polyethylene glycol 12 cetostearyl ether, polyethylene glycol 20 cetostearyl ether, polysorbate 20, polysorbate 60, polysorbate 80, dibutyl sebacate, diethyl phthalate, glycerol, methyl 4-hydroxybenzoate, glycerol, castor oil, oleic acid, glyceryl triacetate, polyalkylene glycol, glyceryl monostearate, stearic acid, acacia, alginate, alginic acid, aluminum acetate, butyl paraben, butylated hydroxytoluene, citric acid, palmitic acid, calcium carbonate, maydelilla wax, croscarmellose sodium, fructose, colloidal silicon dioxide, cellulose, ordinary or anhydrous calcium phosphate, carnauba wax, corn starch, carboxymethylcellulose calcium, calcium disodium ethylenediaminetetraacetate (EDTA), dicalcium hydrogen phosphate dehydrate, cetylpyridine, dicalcium hydrogen phosphate, tricalcium phosphate, dicalcium hydrogen phosphate, disodium hydrogen phosphate, dimethicone, sodium erythrosine, ethylenediaminetetraacetic acid (EDTA), gelatin, glyceryl oleate, iron oxide, iron sesquioxide, iron oxide yellow, iron oxide red, lactose (aqueous, anhydrous, monohydrate, or spray-dried), microcrystalline cellulose, magnesium carbonate, magnesium oxide, methyl paraben, polysorbate-80, propyl paraben, potassium bicarbonate, potassium sorbate, potato starch, phosphoric acid, polyoxyethylene (40) stearate, sodium arc glycolic acid, pregelatinized starch, croscamellose sodium, sodium dodecyl sulfate, starch, silicon dioxide, sodium benzoate, sucrose, sorbic acid, sodium carbonate, sodium saccharin, sodium alginate, silica gel, sorbitan monooleate, sodium chloride, sodium metabisulfite, sodium citrate dehydrate, sodium starch, sodium carboxymethylcellulose, succinic acid, sodium propionate, titanium dioxide, talc, magnesium stearate, and calcium stearate.

In some embodiments, the plug or intermediate layer is printed by hot-melt extrusion of the material.

### Shell

In some embodiments, the pharmaceutical unit comprises a shell. In some embodiments, the shell partially surrounds the drug-containing inner core.

In some embodiments, the shell is not mixed with a drug. In some embodiments, the shell is mixed with different drugs.

In some embodiments, the shell is not erodible. In some embodiments, the shell has a slower erosion rate than the ER layer. In some embodiments, the shell is not substantially eroded until substantially all of the drugs in the ER layer has been released therefrom. In some embodiments, the shell is substantially not eroded for a period of at least about 6 h (such as any one of at least about 7 h, 8 h, 9 h, 10 h, 11 h, 12 h, 14 h, 16 h, 18 h, 20 h, 22 h, 22 h, 24 h, 30 h, 36 h, 48 h, and 72 h) after administration of the dosage unit to an individual. In some embodiments, the shell comprises a pH-sensitive material, such as a material that is eroded within a particular pH range.

In some embodiments, the shell is impermeable, for example, to water or gastrointestinal fluids. In some embodiments, the shell is substantially impermeable.

In some embodiments, the material of the shell portion may include a thermoplastic polymer, which may be any one or a combination of the materials listed below: cellulose acetate phthalate (CAP), poly[butyl methacrylate, (2-dimethylaminoethyl)methyl methacrylate, methyl methacrylate] in a ratio of 1:2:1 (e.g., EUDRAGIT^{®}E 100, EUDRAGIT^{®}E 12,5, and EUDRAGIT^{®}E PO), poly[ethyl acrylate, methyl methacrylate] in a ratio of 2:1 (e.g., EUDRAGIT^{®}NE 30 D, EUDRAGIT^{®}NE 40 D, and EUDRAGIT^{®}NM 30 D), poly [methacrylic acid, methyl methacrylate] in a ratio of 1:1 (e.g., EUDRAGIT^{®}L 100, EUDRAGIT ^{®}L 12,5, and EUDRAGIT^{®}L 12,5 P), poly [methacrylic acid, ethyl acrylate] in a ratio of 1:1 (e.g., Acryl-EZE, Acryl-EZE 93A, Acryl-EZE MP, EUDRAGIT^{®}L 30 D -55, EUDRAGIT^{®}L 100-55, Eastacryl^{®}30 D, Kollicoat^{®}MAE 30 DP, and Kollicoat^{®}MAE 100 P), poly [methacrylic acid, methyl methacrylate] in a ratio of 1:2 (e.g., EUDRAGIT^{®}S 100, EUDRAGIT ^{®}S 12,5, and EUDRAGIT^{®}12,5 P), poly[methyl acrylate, methyl methacrylate, methacrylic acid] in a ratio of 7:3:1 (e.g., EUDRAGIT^{®}FS 30 D), poly[ethyl acrylate, methyl methacrylate, chlorotrimethylammoniumethyl methacrylate] in a ratio of 1:2:0.2 (e.g., EUDRAGIT^{®}RL 100, EUDRAGIT^{®} RL PO, EUDRAGIT^{®}RL 30 D, and EUDRAGIT^{®}RL 12,5), poly[ethyl acrylate, methyl methacrylate, chlorotrimethylammoniumethyl methacrylate] in a ratio of 1:2:0.1 (e.g. EUDRAGIT^{®}RS 100, EUDRAGIT^{®}RS PO, EUDRAGIT^{®}RS 30 D, and EUDRAGIT^{®}RS 12,5), poly(lactic-co-glycolic acid) copolymer (PLGA), ethylene-vinyl acetate copolymer (EVA), polyethylene (PE), polycaprolactone (PCL), polylactic acid (PLA), cellulose acetate butyrate (CAB), cellulose acetate (CA), polyvinyl acetate (PVAc), polyvinylacetal diethylamino acetate (AEA), ethylcellulose (EC), carboxymethylethylcellulose (CMCE), carbomer, phospholipid, and cyclodextrin.

In some embodiments, the material of the shell portion may further include an expanding material, which includes any one or a combination of the materials listed below: high molecular weight hydroxypropylcellulose (HPC), high molecular weight hydroxypropyl methylcellulose or hypromellose (HPMC), methylcellulose (MC), high molecular weight polyethylene oxide (PEO), high molecular weight polyvinyl alcohol (PVA), polyvinyl acetate (PVAc) and polyvinylpyrrolidone (PVP) 80/20, poly[butyl methacrylate, (2-dimethylaminoethyl)methyl methacrylate, methyl methacrylate] in a ratio of 1:2:1 (e.g., EUDRAGIT^{®}E 100, EUDRAGIT^{®}E 12,5, and EUDRAGIT^{®}E PO), poly[ethyl acrylate, methyl methacrylate] in a ratio of 2:1 (e.g., EUDRAGIT^{®}NE 30 D, EUDRAGIT^{®}NE 40 D, and EUDRAGIT^{®}NM 30 D), poly [methacrylic acid, methyl methacrylate] in a ratio of 1:1 (e.g., EUDRAGIT^{®}L 100, EUDRAGIT ^{®}L 12,5, and EUDRAGIT^{®}L 12,5 P), poly [methacrylic acid, ethyl acrylate] in a ratio of 1:1 (e.g., Acryl-EZE, Acryl-EZE 93A, Acryl-EZE MP, EUDRAGIT^{®}L 30 D -55, EUDRAGIT^{®}L 100-55, Eastacryl^{®}30 D, Kollicoat^{®}MAE 30 DP, and Kollicoat^{®}MAE 100 P), poly[methacrylic acid, methyl methacrylate] in a ratio of 1:2 (e.g., EUDRAGIT^{®}S 100, EUDRAGIT ^{®}S 12,5, and EUDRAGIT^{®}12,5 P), poly[methyl acrylate, methyl methacrylate, methacrylic acid] in a ratio of 7:3:1 (e.g., EUDRAGIT^{®}FS 30 D), poly[ethyl acrylate, methyl methacrylate, chlorotrimethylammoniumethyl methacrylate] in a ratio of 1:2:0.2 (e.g., EUDRAGIT^{®}RL 100, EUDRAGIT^{®} RL PO, EUDRAGIT^{®}RL 30 D, and EUDRAGIT^{®}RL 12,5), poly[ethyl acrylate, methyl methacrylate, chlorotrimethylammoniumethyl methacrylate] in a ratio of 1:2:0.1 (e.g. EUDRAGIT^{®}RS 100, EUDRAGIT^{®}RS PO, EUDRAGIT^{®}RS 30 D, and EUDRAGIT^{®}RS 12,5), hydroxypropyl methylcellulose acetate succinate (HPMCAS), hydroxypropyl methylcellulose phthalate (HPMCP), polyvinylacetal diethylamino lactate (AEA), and a combination thereof.

In some embodiments, the dissolution of the shell is pH-dependent. In some embodiments, the dissolution of the shell occurs at a pH above about 5.5 to about 7. In some embodiments, the erosion occurs at a pH above about 5.5, about 6, about 6.5, or about 7. In some embodiments, the shell comprises an enteric material.

In some embodiments, the shell is printed by hot-melt extrusion of the shell material.

The "pharmaceutical unit" structure and material described herein may be any pharmaceutical unit structure, compartment structure, intermediate layer structure, dosage unit structure, and a combination thereof described in U.S. Patent Nos. US10258575 and US 10363220, U.S. Patent Application Nos. US2019192440, US2019321299, and US2020315971, and International Patent Application Publication Nos. WO2019137200, WO2016192680, WO2017193099, WO2018137686, and WO2019137199, and their corresponding national stage patent applications. The patents and patent applications described above are incorporated herein by reference in their entirety.

It should be noted that in some embodiments, the shell portion of the pharmaceutical unit may be made of the same material as the unfolding member, thereby effectively improving the manufacturing efficiency of the pharmaceutical dosage form.

As used herein, the "drug" includes, but is not limited to, a drug, a prodrug, a biological formulation, and any other substance that may be administered to inhibit a disease or bring about a beneficial health effect.

In some embodiments, the drug in the pharmaceutical unit 102 is a drug administered through the gastrointestinal tract, which may be one or a combination of the drugs selected from the following: statins, such as rosuvastatin; non-steroidal anti-inflammatory drugs (NSAIDs), such as meloxicam; blood diluents, such as clopidogrel; analgesics, such as buprenorphine; opioid antagonists, such as naloxone; anti-asthma agents, such as montelukast; steroids, such as prednisone; antipsychotic agents, such as aripiprazole and risperidone; cardiac glycosides, such as digoxin; α-blockers, such as tamsulosin; cholesterol absorption inhibitors, such as ezetimibe; antihistamines, such as loratadine and cetirizine; proton pump inhibitors, such as omeprazole; antiviral agents, such as entecavir; antibiotics, such as doxycycline, ciprofloxacin, and azithromycin; substance abuse therapeutic agents, such as methadone and varenicline; contraceptive agents; stimulants, such as caffeine; and antigout therapeutic agents, such as colchicine. In some embodiments, the drug base may be nutrients, including but not limited to: folic acid, calcium, iodine, iron, zinc, nicotinic acid, vitamin C, vitamin D, biotins, plant extracts, phytohormones, and other vitamins or minerals.

### Retaining Member

A retaining member is connected to at least one unfolding member and applies a constraint force to the at least one unfolding member to cause the unfolding member to be in a contracted shape, wherein the retaining member comprises a water-soluble material and is configured to remove the constraint force from the unfolding member when the water-soluble material is dissolved, thereby enabling the at least one unfolding member to change from the contracted shape to an unfolding shape for effective retention in the gastrointestinal tract. In some embodiments, particularly when the constraint force is applied from the outside of the unfolding member, the retaining member should generally have a relatively high rigidity in order to achieve the constraint force against the unfolding member.

In some embodiments, the retaining member material is a thermoplastic material. In some embodiments, the water-soluble immediate-release thermoplastic material is selected from one or a combination of: polyvinyl caprolactam-polyvinyl acetate-polyethylene glycol graft copolymer 57/30/13, vinylpyrrolidone-vinyl acetate copolymer (PVP-VA), vinylpyrrolidone-vinyl acetate copolymer (PVP-VA) 60/40, polyvinylpyrrolidone (PVP), polyvinyl acetate (PVAc) and polyvinylpyrrolidone (PVP) 80/20, vinylpyrrolidone-vinyl acetate copolymer (VA64), polyethylene glycol-polyvinyl alcohol graft copolymer 25/75, kollicoat IR-polyvinyl alcohol 60/40, polyvinyl alcohol (PVA or PV-OH), polyethylene oxide (PEO), polyethylene glycol (PEG), cellulose or cellulose derivatives, hydroxypropyl methylcellulose acetate succinate or hypromellose succinate (HPMCAS), carbomer, hydroxypropylcellulose (HPC), poloxamer, hydroxypropyl methylcellulose phthalate (HPMCP), poloxamer, polyglycolic acid (PGA), saccharides, glucose, hydrogel, gelatin, sodium alginate, gum arabic, and xanthan gum.

In some embodiments, the retaining member material further comprises a plasticizer or other additives selected from one or a combination of: triethyl citrate (TEC), vitamin E polyethylene glycol succinate (TPGS), acetin, acetylated triethyl citrate, tributyl citrate, acetylated tributyl citrate, polyhydroxy 15 hydroxystearate, polyoxyethylene-40 hydrogenated castor oil, polyoxyethylene-35 castor oil, polyoxyethylene-polyoxypropylene block copolymer, dibutyl sebacate, diethyl phthalate, glycerol, methyl 4-hydroxybenzoate, castor oil, oleic acid, triacetin, polyalkylene glycol, hydroxy stearate, polyethylene glycol (e.g., PEG400), polyethylene glycol 12 cetostearyl ether, polyethylene glycol 20 cetostearyl ether, polysorbate 20, polysorbate 60, polysorbate 80, dibutyl sebacate, diethyl phthalate, glycerol, methyl 4-hydroxybenzoate, glycerol, castor oil, oleic acid, glyceryl triacetate, polyalkylene glycol, glyceryl monostearate, stearic acid, acacia, alginate, alginic acid, aluminum acetate, butyl paraben, butylated hydroxytoluene, citric acid, palmitic acid, calcium carbonate, maydelilla wax, croscarmellose sodium, fructose, colloidal silicon dioxide, cellulose, ordinary or anhydrous calcium phosphate, carnauba wax, corn starch, carboxymethylcellulose calcium, calcium disodium ethylenediaminetetraacetate (EDTA), dicalcium hydrogen phosphate dehydrate, cetylpyridine, dicalcium hydrogen phosphate, tricalcium phosphate, dicalcium hydrogen phosphate, disodium hydrogen phosphate, dimethicone, sodium erythrosine, ethylenediaminetetraacetic acid (EDTA), gelatin, glyceryl oleate, iron oxide, iron sesquioxide, iron oxide yellow, iron oxide red, lactose (aqueous, anhydrous, monohydrate, or spray-dried), microcrystalline cellulose, magnesium carbonate, magnesium oxide, methyl paraben, polysorbate-80, propyl paraben, potassium bicarbonate, potassium sorbate, potato starch, phosphoric acid, polyoxyethylene (40) stearate, sodium arc glycolic acid, pregelatinized starch, croscamellose sodium, sodium dodecyl sulfate, starch, silicon dioxide, sodium benzoate, sucrose, sorbic acid, sodium carbonate, sodium saccharin, sodium alginate, silica gel, sorbitan monooleate, sodium chloride, sodium metabisulfite, sodium citrate dehydrate, sodium starch, sodium carboxymethylcellulose, succinic acid, sodium propionate, titanium dioxide, talc, magnesium stearate, and calcium stearate.

In other embodiments, the retaining member may be located at least partially between the unfolding member and the pharmaceutical unit to bond them, thereby retaining the unfolding member in the contracted shape; at this time, the retaining member may be partially exposed from the surface of the pharmaceutical dosage form, thereby making the liquid to dissolve the retaining member and eventually remove the bond between the unfolding member and the pharmaceutical unit.

In some embodiments, the retaining member at least partially coats the pharmaceutical unit and the at least one unfolding member, such that the at least one unfolding member is in a contracted shape.

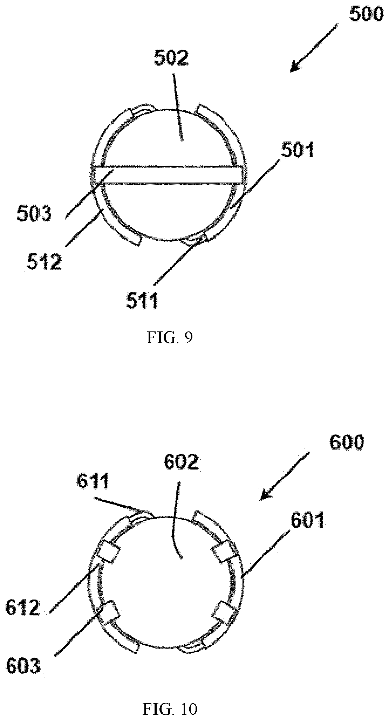

FIGs. 9 and 10 show schematic top views of pharmaceutical dosage forms 500 and 600, respectively, according to embodiments of the present application. The pharmaceutical dosage forms 500 and 600 have structures substantially similar to the pharmaceutical unit and the unfolding member of a pharmaceutical dosage form 100 shown in FIGs. 1 and 2. As shown in FIG. 9, an elongated retaining member 503 is located on an unfolding member 501 and a pharmaceutical unit 502 and is connected to them simultaneously, so as to retain them in a contracted shape where the unfolding member 501 is fitted around the outer peripheral wall of the pharmaceutical unit 502 as shown in the figure. As shown in FIG. 10, the retaining member 503 may also be a plurality of dot-block structures that are located on the unfolding member 501 and the pharmaceutical unit 502 and discretely attached along a gap where the unfolding member and the pharmaceutical unit are fitted against each other. In some embodiments, in order to provide a reliable constraint effect, the retaining member 503 may also be a ring structure attached along an upper portion of a joint gap of the unfolding member 501 and the pharmaceutical unit 502.

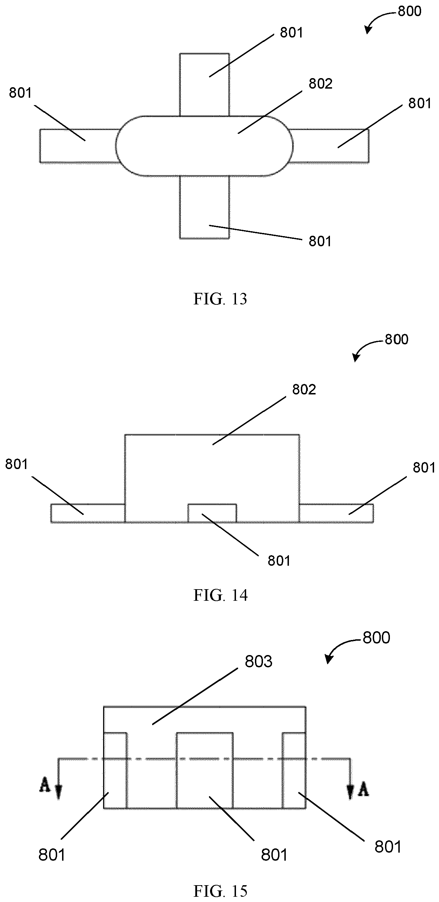

FIGs. 13-16 show a top view and a front view of a pharmaceutical dosage form 800 according to one embodiment of the present application in an unfolding shape after a retaining member is removed, and a front view and a cross-sectional top view of the pharmaceutical dosage form. As shown in the figures, when in a contracted shape, unfolding members 801 are folded upwards to fit and cover a portion of the outer peripheral sidewall of a pharmaceutical unit 802, and the retaining member covers not only the other portions of the outer peripheral sidewall of the pharmaceutical unit 802, but also an upper portion connecting the unfolding members 801 and the pharmaceutical unit 802 simultaneously, thereby providing an effective and reliable constraint. In some other embodiments, a retaining member 803 may also cover only the outer peripheral sidewall of the pharmaceutical unit 802 without covering the unfolding member 801 and the upper or lower portion of the pharmaceutical unit 802.

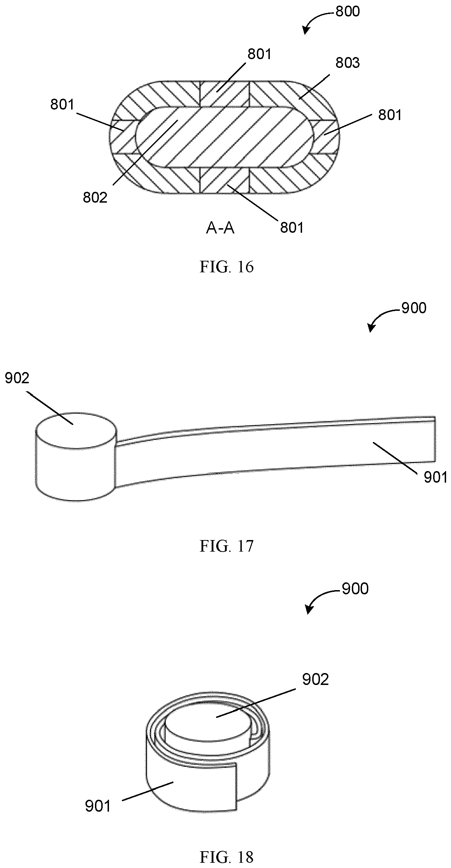

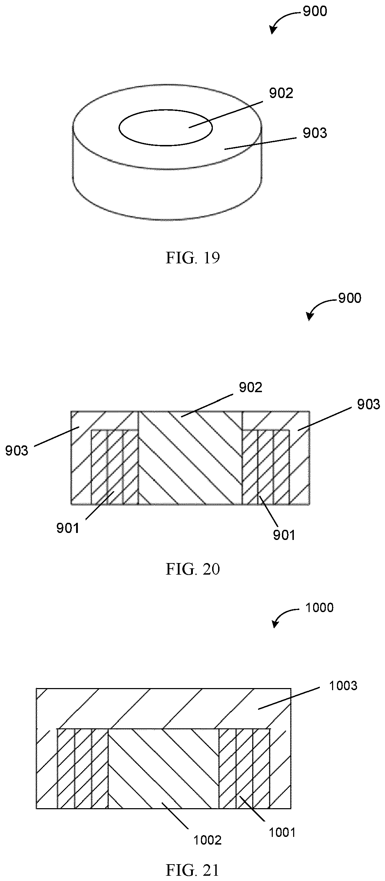

FIGs. 17-20 show schematic perspective views of a pharmaceutical dosage form 900 according to one embodiment of the present application in an unfolding shape and a contracted shape, respectively, after a retaining member is removed, and a schematic perspective view and a schematic cross-sectional view of the pharmaceutical dosage form 900. As shown in FIGs. 17 and 18, an unfolding member 901 of the pharmaceutical dosage form 900 is a strip-shaped member extending from the outer peripheral wall of a pharmaceutical unit 902, and when in the contracted shape, the unfolding member is wound around the outer peripheral sidewall of the pharmaceutical unit 902, and partially covers the outer peripheral sidewall of the pharmaceutical unit 902. As shown in FIGs. 19 and 20, a retaining member 903 coats the other portions of the outer peripheral sidewall of the pharmaceutical unit 902, and covers the upper portion and the outer peripheral sidewall of the unfolding member 901 in the contracted shape. The structure of the embodiment 1000 shown in FIG. 21 is substantially similar to that of the embodiment 900, wherein an unfolding member 1001 wound around a pharmaceutical unit 1002 substantially covers the outer peripheral sidewall of the cylindrical pharmaceutical unit 1002, and a retaining member 1003 completely coats the upper wall and the outer peripheral sidewall of a structure formed by the pharmaceutical unit 1002 and the unfolding member 1001 that is in the contracted shape. In other embodiments, the retaining member 1003 may also completely coat the integral structure formed by the pharmaceutical unit 1002 and the unfolding member 1001 that is in the contracted shape.

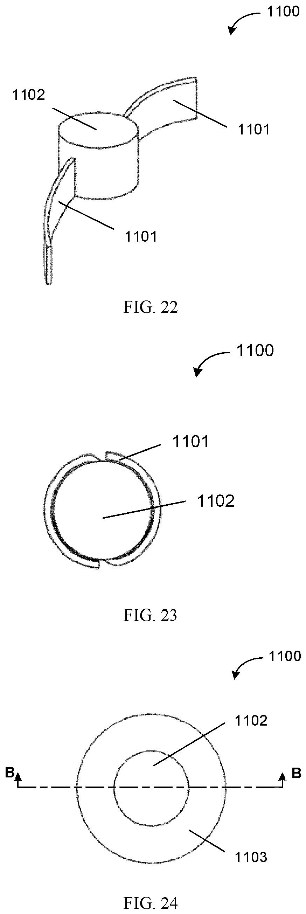

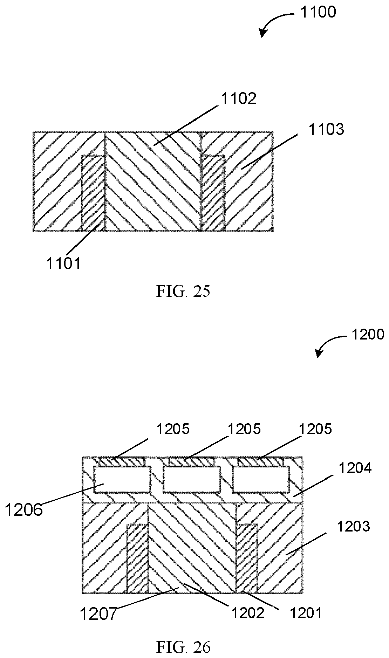

FIGs. 22-25 show schematic perspective views of a pharmaceutical dosage form 1100 according to one embodiment of the present application in an unfolding shape and a contracted shape, respectively, after a retaining member is removed, and a schematic top view and a schematic cross-sectional view of the pharmaceutical dosage form 1100. As shown in FIGs. 22 and 23, unfolding members 1101 of the pharmaceutical dosage form 1100 are two strip-shaped members on the outer peripheral wall of a pharmaceutical unit 1102, and when in the contracted shape, the two unfolding members 1101 are wound around the outer peripheral sidewall of the pharmaceutical unit 1102, respectively, and partially covers the outer peripheral sidewall of the pharmaceutical unit 1102. As shown in FIGs. 24 and 25, a retaining member 1103 coats the other portions of the outer peripheral sidewall of the pharmaceutical unit 1102, and covers the upper portion and the outer peripheral sidewall of the two unfolding members 1101 in the contracted shape. In other embodiments, the retaining member 1103 may also completely coat a structure formed by the pharmaceutical unit 1102 and the unfolding members 1101 that is in the contracted shape, or coats the upper wall and the outer peripheral sidewall of the structure.

In some embodiments, the pharmaceutical unit may comprise a drug-containing portion and a drug-free portion, and the drug-containing portion is configured to be formed independently of other portions.

The structures of unfolding members 1201, a pharmaceutical unit 1204 and a retaining member 1203 of a pharmaceutical dosage form 1200 as shown in FIG. 26 are substantially similar to those of Example 1. The difference is that a floating member 1207 is attached to the illustrated pharmaceutical unit 1204. The pharmaceutical unit 1204 defines three compartments 1206, each of which may be filled with a drug-containing inner core material as shown above, such as a drug or a mixture of a drug and an erodible material or other excipients, or the like. The shell material of the pharmaceutical unit 1204 may be the material used in the shell portion described above, and the upper portion of the pharmaceutical unit is provided with plug structures 1205 described above to be eroded in the gastrointestinal fluid, so that the drug in the compartments may be exposed to the gastrointestinal fluid. In some embodiments, the three plug structures 1205 as shown in FIG. 26 may be made of materials with different dissolution rates or provided with different dimensions (e.g., thickness, size, etc.) to achieve a pulsed release of the drug-containing inner core material within the compartments 1206. In some embodiments, the floating member 1207 may be configured as a cavity, honeycomb, or scaffold structure, thereby providing buoyancy to achieve a better retention effect. The pharmaceutical unit 1204 described above may be formed independently of other portions by the three-dimensional printing or additive manufacturing method. Although FIG. 26 shows a specific structure of the pharmaceutical unit 1204, it may also be any available 3D-printed pharmaceutical dosage form configuration. Since the drug-containing portion may be printed independently of other structures, for example, the portions under the pharmaceutical unit 1204 are first printed, and then the pharmaceutical unit 1204 is printed independently, therefore, the pharmaceutical unit 1204 may adopt any existing procedure, method, or structural design for manufacturing a pharmaceutical dosage form by printing or additive manufacturing without further modification to meet the gastric retention requirements, thereby reducing the development and manufacturing costs of a specific product.

For example, in some embodiments, a single compartment of the pharmaceutical unit 1204 may be provided with a plurality of drug layers made of erodible materials with different dissolution rates, and different combinations of stacking positions, shapes, and sizes of the drug layers may achieve a target release profile for one or more drugs. In other embodiments, the number of the compartments may be any other number, such as 1, 2, 4, 5, 6, etc. In some embodiments, the plurality of compartments may be filled with the same or different drug materials, and may have different positional relationships, such as stacked, staggered, and the like. In other embodiments, some of the compartments may be left empty, so that they may provide additional buoyancy for the pharmaceutical dosage form to achieve a better retention effect.

Although the pharmaceutical unit 1204 as shown in the figure is connected to the floating member 1207 and the retaining member 1203, in other embodiments, the pharmaceutical unit 1204 may also be connected to the unfolding members 1201 simultaneously, or only to either of the floating member 1207 and the unfolding members 1201, to achieve effective retention of the pharmaceutical unit 1204 in the gastrointestinal tract space with the assistance of the unfolding members 1201 in the unfolding shape.

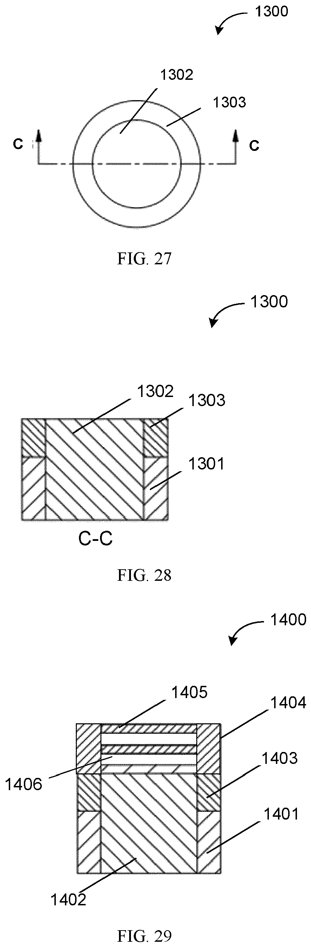

FIGs. 27 and 28 show a schematic top view and a schematic cross-sectional view of a pharmaceutical dosage form 1300 according to one embodiment of the present application. The pharmaceutical dosage form 1300 has a similar configuration to the pharmaceutical unit and the unfolding members as shown in FIGs. 22 and 23, which will not be described herein again. As shown in FIGs. 27 and 28, unfolding members 1301 substantially coat the lower half portion of the sidewall of a pharmaceutical unit 1302 when in a contracted shape, while a retaining member 1303 coats the upper half portion of the outer peripheral sidewall attached to the pharmaceutical unit 1302 and at the same time the retaining member is connected to the upper side of the unfolding members 1301 at the lower side thereof, thereby retaining the unfolding members 1301 in the contracted shape. The above arrangement may minimize the size of the final pharmaceutical dosage form and reduce the cost while maintaining the constraint effect on the unfolding member.

The structures of unfolding members 1401, a floating member 1407 and a retaining member 1403 of a pharmaceutical dosage form 1400 as shown in FIG. 29 are substantially similar to those of Example 3. The difference is that the illustrated pharmaceutical unit consists of an outer shell 1404, drug-containing layers 1406, and a plug or intermediate layer 1405. The pharmaceutical unit defines two stacked compartments 1406 therein, and the compartments may be filled with drug layers or materials similar to those of the compartments 1206, which will not be described herein again. The upper portion of the compartments is provided with a cap structure 1405 similar to the cap structure 1205 on the pharmaceutical dosage form 1200. Similar to the structure of the drug-containing portion 1204 shown in FIG. 28 above, the pharmaceutical unit of the pharmaceutical dosage form 1400 shown in FIG. 29 may also be formed independently of other portions. Thus, the pharmaceutical unit may also be designed by adopting existing well-established 3D-printed pharmaceutical dosage form designs or manufacturing programs.

In some embodiments, the retaining member is disposed between the pharmaceutical unit and the unfolding member, and is connected to the pharmaceutical unit, such that the unfolding member is in a contracted shape. Specifically, in some embodiments, there are gaps between the unfolding member in the contracted shape and the pharmaceutical unit, and the retaining member is used to at least partially fill these gaps and cause the unfolding member to be in the contracted shape. In some embodiments, the pharmaceutical dosage form comprises a plurality of unfolding members, and the retaining member is connected to at least two unfolding members, such that at least two unfolding members are in the contracted shape.

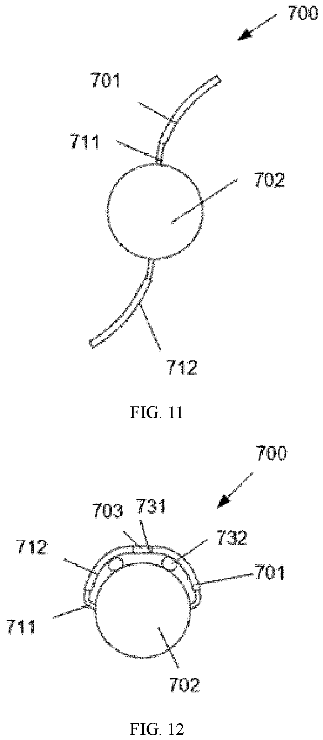

FIG. 12 shows a schematic top view of a pharmaceutical dosage form 700 according to one embodiment of the present application. FIG. 11 shows a schematic top view of the pharmaceutical dosage form 700 with unfolding members 701 in an unfolding shape after a retaining member is removed. The pharmaceutical dosage form 700 shown in FIG. 11 has a structure in the unfolding shape substantially similar to that of the embodiment shown in FIG. 1, with two unfolding members 701 extending from a cylindrical pharmaceutical unit 702. A retaining member 703 of the pharmaceutical dosage form 700 as shown in FIG. 12 includes a plurality of portions, and the ends of the two unfolding members 701 distal to the pharmaceutical unit 702 adjacent to each other are connected to each other by a retaining member 731 to constrain the two unfolding members 701 in the contracted state. In addition, the retaining member 703 of the pharmaceutical dosage form 700 further includes two retaining members 732 as shown in the figure, the retaining members 732 being disposed between the unfolding members 701 and the pharmaceutical unit 702 to assist in retaining the unfolding members 701 in the contracted shape. It will be understood that the retaining members 732 may be exposed from at least a portion of an outer surface of the pharmaceutical dosage form 700, thereby allowing the liquid to contact the retaining members 732 to gradually dissolve the retaining members 732 when the pharmaceutical dosage form 700 is placed in an aqueous solution.

It should be noted that although FIG. 12 shows that the retaining member 703 includes two types of retaining members 731 and 732, in some embodiments, the retaining member may also include only one of them. In other embodiments, the retaining member 703 may also adopt a combination of several retaining member arrangement forms described above.

### Pharmaceutical Dosage Form II

The present application further provides another pharmaceutical dosage form. The pharmaceutical dosage form comprises a pharmaceutical unit comprising a drug and a retaining member, wherein at least a portion of the pharmaceutical unit is in a bent or folded shape, while the retaining member is connected to the pharmaceutical unit and applies a constraint force to the pharmaceutical unit such that at least a portion of the pharmaceutical unit retains the bent or folded shape. The retaining member comprises a water-soluble material and is configured to remove the constraint force from the pharmaceutical unit when the water-soluble material is dissolved, thereby allowing at least a portion of the pharmaceutical unit to unfold from the bent or folded shape. In some embodiments, the pharmaceutical dosage form may be formed by printing with a 3D printing method.

The pharmaceutical dosage form does not need to be provided with a complex structure of an unfolding member, and after the retaining member is at least partially dissolved in the gastrointestinal fluid, the pharmaceutical unit itself may be unfolded from the bent or folded shape to expand its whole size, so that the retention of the pharmaceutical dosage form in the stomach or other parts of the upper digestive tract is achieved, the retention time of the drug in the part to be applied is prolonged, the dosing frequency is reduced, and the bioavailability is improved.

In some embodiments, the pharmaceutical unit of the pharmaceutical dosage form may be entirely elastic or comprises an elastic portion as described above in FIGs. 1 and 2, and under the action of the elastic portion, the pharmaceutical unit rapidly returns to an unbent or folded state after losing the constraint of the retaining member to achieve the retention effect. In other embodiments, the pharmaceutical unit of the pharmaceutical dosage form may not comprise an elastic portion or an elastic material, and when the retaining member is eroded, the pharmaceutical unit may naturally unfold into an unbent or folded shape under the action of the gastrointestinal fluid or an external force from the tissues of the gastrointestinal tract. In still other embodiments, the pharmaceutical unit may be provided with different thicknesses, thereby forming at least one portion with reduced thickness as described above. Since no elastic material is used in the above structure, when the pharmaceutical dosage form is printed by a 3D printing technique, the pharmaceutical unit may be printed directly in a contracted shape, which effectively improves the printing efficiency.

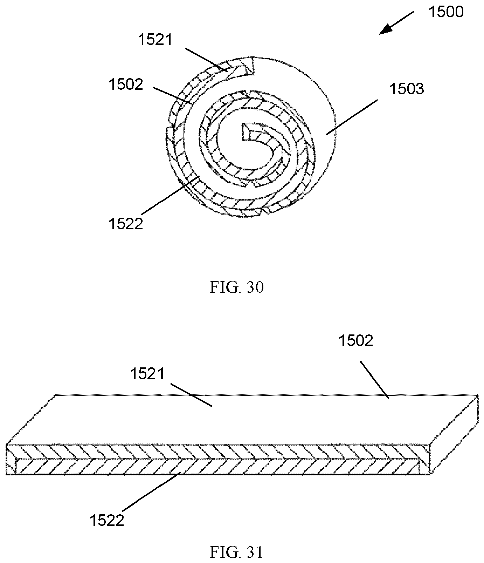

FIG. 30 shows a schematic top view of a pharmaceutical dosage form 1500 according to one embodiment of the present application. FIG. 31 shows a schematic perspective view of the pharmaceutical dosage form 1500 shown in FIG. 30 unfolded in a strip shape after a retaining member is removed. As shown in FIGs. 30 and 31, the pharmaceutical dosage form 1500 comprises a pharmaceutical unit 1502 and a retaining member 1503. The pharmaceutical unit 1502 has a spiral shape with a gradually increasing radius of curvature from the inside to the outside, while the retaining member 1503 of the white area fills the gap of the spiral shape of the pharmaceutical unit 1502.

Although the retaining member 1503 as shown in the figure fills the entire gap of the spiral shape of the pharmaceutical unit 1502, in other embodiments, the retaining member 1503 may only fill a portion of the gap, thereby reducing the cost and improving the production efficiency. Although the pharmaceutical unit 1502 as shown in FIG. 31 is in a strip shape when fully unfolded, in some embodiments, the pharmaceutical unit 1502 may also be configured as an elongated member in other shapes that facilitate bending or folding, such as a string, a rod, or a cuboid shape. In addition, although the pharmaceutical unit 1502 as shown in the figure is coiled in a spiral shape, in other embodiments, the pharmaceutical unit 1502 may be folded in any other shape that may effectively reduce the extension length of the pharmaceutical unit in a certain direction, for example, an S-shaped, 8-shaped, or Z-shaped continuously folded shape, etc.

Referring to FIGs. 30 and 31 again, the pharmaceutical unit 1502 specifically comprises a shell portion 1521 and a drug-containing portion 1522 embedded within a groove formed in the shell. The drug-containing portion 1522 may be composed of a drug, a pharmaceutical excipient, and an erodible material. In some embodiments, the drug-containing portion 1522 may be configured as multiple layers of overlapping drug-containing portions composed of different erosion materials and/or drugs as described above.

In some embodiments, the shell portion 1521 is made of a hydrophobic material or a pH-dependent material, so that it may provide buoyancy or surface tension for the pharmaceutical unit in the gastric juice to achieve a better retention effect. In other embodiments, an outer surface of the shell portion 1521 is provided with a hydrophobic material or a pH-dependent material. Specifically, the hydrophobic material or the PH-dependent material may be selected from one or more of the following pharmaceutical grade polymers: cellulose acetate phthalate (CAP), poly[butyl methacrylate, (2-dimethylaminoethyl)methyl methacrylate, methyl methacrylate] in a ratio of 1:2:1 (e.g., EUDRAGIT^{®}E 100, EUDRAGIT^{®}E 12,5, and EUDRAGIT^{®}E PO), poly[ethyl acrylate, methyl methacrylate] in a ratio of 2:1 (e.g., EUDRAGIT^{®}NE 30 D, EUDRAGITONE 40 D, and EUDRAGITONM 30 D), poly [methacrylic acid, methyl methacrylate] in a ratio of 1:1 (e.g., EUDRAGIT^{®}L 100, EUDRAGIT ^{®}L 12,5, and EUDRAGIT^{®}L 12,5 P), poly [methacrylic acid, ethyl acrylate] in a ratio of 1:1 (e.g., Acryl-EZE, Acryl-EZE 93A, Acryl-EZE MP, EUDRAGIT^{®}L 30 D -55, EUDRAGIT^{®}L 100-55, Eastacryl^{®}30 D, Kollicoat^{®}MAE 30 DP, and Kollicoat^{®}MAE 100 P), poly[methacrylic acid, methyl methacrylate] in a ratio of 1:2 (e.g., EUDRAGIT^{®}S 100, EUDRAGIT ^{®}S 12,5, and EUDRAGIT^{®}12,5 P), poly[methyl acrylate, methyl methacrylate, methacrylic acid] in a ratio of 7:3:1 (e.g., EUDRAGIT^{®}FS 30 D), poly[ethyl acrylate, methyl methacrylate, chlorotrimethylammoniumethyl methacrylate] in a ratio of 1:2:0.2 (e.g., EUDRAGIT^{®}RL 100, EUDRAGIT^{®} RL PO, EUDRAGIT^{®}RL 30 D, and EUDRAGIT^{®}RL 12,5), poly[ethyl acrylate, methyl methacrylate, chlorotrimethylammoniumethyl methacrylate] in a ratio of 1:2:0.1 (e.g. EUDRAGIT^{®}RS 100, EUDRAGIT^{®}RS PO, EUDRAGIT^{®}RS 30 D, and EUDRAGIT^{®}RS 12,5), poly(lactic-co-glycolic acid) copolymer (PLGA), ethylene-vinyl acetate copolymer (EVA), polyethylene (PE), polycaprolactone (PCL), polylactic acid (PLA), cellulose acetate butyrate (CAB), cellulose acetate (CA), polyvinyl acetate (PVAc), polyvinylacetal diethylamino acetate (AEA), ethylcellulose (EC), carboxymethylethylcellulose (CMCE), carbomer, phospholipid, and cyclodextrin.

In other embodiments, the drug-containing portion described above comprises a hydrophilic material, a PH-dependent material, or a hydrophobic material, which may be selected from one or more of the following pharmaceutical grade polymers: copovidone (polyvinylpyrrolidone-vinyl acetate copolymer (PVP-VA) and polyvinylpyrrolidone-polyvinyl acetate copolymer (PVP-VA) 60/40), crospovidone, polyvinylpyrrolidone (PVP), hydroxypropylcellulose (HPC), hydroxypropyl methylcellulose or hypromellose (HPMC), hydroxypropyl methylcellulose phthalate (HPMCP), methylcellulose (MC), methacrylate copolymer, poly[butyl methacrylate, (2-dimethylaminoethyl)methyl methacrylate, methyl methacrylate] in a ratio of 1:2:1 (e.g., EUDRAGIT^{®}E 100, EUDRAGIT^{®}E 12,5, and EUDRAGIT^{®}E PO), poly[ethyl acrylate, methyl methacrylate] in a ratio of 2:1 (e.g., EUDRAGIT^{®}NE 30 D, EUDRAGIT^{®}NE 40 D, and EUDRAGIT^{®}NM 30 D), poly [methacrylic acid, methyl methacrylate] in a ratio of 1:1 (e.g., EUDRAGIT^{®}L 100, EUDRAGIT ^{®}L 12,5, and EUDRAGIT^{®}L 12,5 P), poly [methacrylic acid, ethyl acrylate] in a ratio of 1:1 (e.g., Acryl-EZE, Acryl-EZE 93A, Acryl-EZE MP, EUDRAGIT^{®}L 30 D -55, EUDRAGIT^{®}L 100-55, Eastacryl^{®}30 D, Kollicoat^{®}MAE 30 DP, and Kollicoat^{®}MAE 100 P), poly [methacrylic acid, methyl methacrylate] in a ratio of 1:2 (e.g., EUDRAGIT^{®}S 100, EUDRAGIT ^{®}S 12,5, and EUDRAGIT^{®}12,5 P), poly[methyl acrylate, methyl methacrylate, methacrylic acid] in a ratio of 7:3:1 (e.g., EUDRAGIT^{®}FS 30 D), poly[ethyl acrylate, methyl methacrylate, chlorotrimethylammoniumethyl methacrylate] in a ratio of 1:2:0.2 (e.g., EUDRAGIT^{®}RL 100, EUDRAGIT^{®} RL PO, EUDRAGIT^{®}RL 30 D, and EUDRAGIT^{®}RL 12,5), poly[ethyl acrylate, methyl methacrylate, chlorotrimethylammoniumethyl methacrylate] in a ratio of 1:2:0.1 (e.g. EUDRAGIT^{®}RS 100, EUDRAGIT^{®}RS PO, EUDRAGIT^{®}RS 30 D, and EUDRAGIT^{®}RS 12,5), polyethylene oxide (PEO), polyethylene glycol (PEG), polyvinyl caprolactam-polyvinyl acetate-polyethylene glycol graft copolymer 57/30/13, polyethylene glycol-polyvinyl alcohol graft copolymer 25/75 (Kollicoat^{®} IR), Kollicoat^{®} IR-polyvinyl alcohol 60/40 (Kollicoat^{®} Protect), polyvinyl alcohol (PVA), hydroxypropyl methylcellulose succinate or hypromellose acetate succinate (HPMCAS), ethylcellulose (EC), polyvinyl acetate (PVAc) and polyvinylpyrrolidone (PVP) 80/20, polyvinylacetal diethylamino lactate (AEA), carboxymethylethylcellulose (CMCE), carbomer, phospholipid, and cyclodextrin.

In some embodiments, the material of the drug-containing layer may further comprise a plasticizer and other additives, wherein the plasticizer may be selected from any one or a combination of the materials listed below: triethyl citrate (TEC), vitamin E polyethylene glycol succinate (TPGS), acetin, acetylated triethyl citrate, tributyl citrate, tributyl o-acetyl citrate, polyhydroxy 15 hydroxystearate, polyoxyethylene-40 hydrogenated castor oil, polyoxyethylene-35 castor oil, dibutyl sebacate, diethyl phthalate, glycerol, methyl 4-hydroxybenzoate, castor oil, oleic acid, triacetin, and polyalkylene glycol. The other additives may be any one or a combination of the materials listed below: acacia, alginate, alginic acid, aluminum acetate, butyl paraben, butylated hydroxytoluene, citric acid, calcium carbonate, maydelilla wax, croscarmellose sodium, fructose, colloidal silicon dioxide, cellulose, ordinary or anhydrous calcium phosphate, carnauba wax, corn starch, carboxymethylcellulose calcium, calcium disodium ethylenediaminetetraacetate (EDTA), dicalcium hydrogen phosphate dehydrate, cetylpyridine, dicalcium hydrogen phosphate, tricalcium phosphate, dicalcium hydrogen phosphate, disodium hydrogen phosphate, dimethicone, sodium erythrosine, ethylenediaminetetraacetic acid (EDTA), gelatin, glyceryl oleate, iron oxide, iron sesquioxide, iron oxide yellow, iron oxide red, lactose (aqueous, anhydrous, monohydrate, or spray-dried), microcrystalline cellulose, magnesium carbonate, magnesium oxide, methyl paraben, polysorbate-80, propyl paraben, potassium bicarbonate, potassium sorbate, potato starch, phosphoric acid, polyoxyethylene (40) stearate, sodium arc glycolic acid, pregelatinized starch, croscamellose sodium, sodium dodecyl sulfate, starch, silicon dioxide, sodium benzoate, sucrose, sorbic acid, sodium carbonate, sodium saccharin, sodium alginate, silica gel, sorbitan monooleate, sodium chloride, sodium metabisulfite, sodium citrate dehydrate, sodium starch, sodium carboxymethylcellulose, succinic acid, sodium propionate, titanium dioxide, and talc.

The pharmaceutical dosage form 1500 as shown in FIGs. 30 and 31 is substantially in a disk structure. In some embodiments, the disk structure has a diameter of no more than 22 mm and a thickness of no more than 10 mm. In some embodiments, the pharmaceutical unit 1502 has a maximum length in an unfolding size of greater than 12 mm, and preferably greater than 18 mm.

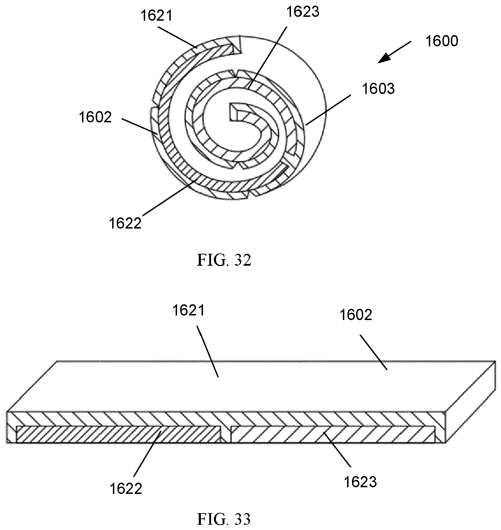

FIG. 32 shows a schematic top view of a pharmaceutical dosage form 1600 according to one embodiment of the present application. FIG. 33 shows a schematic perspective view of the pharmaceutical dosage form 1600 shown in FIG. 32 unfolded in a strip shape after a retaining member is removed. A shell portion 1621 and a retaining member portion of the pharmaceutical dosage form 1600 are similar to those of the pharmaceutical dosage form 1500. The difference is that the shell portion 1621 of the pharmaceutical unit 1602 defines two grooves into which drug-containing portions 1622 and 1623 are respectively embedded on the shell. In some embodiments, the drug-containing portions 1622 and 1623 have different drugs. In other embodiments, the drugs in the drug-containing portions 1622 and 1623 are mixed with materials having different erosion rates, respectively, such that the ratio of the drug-containing portions 1622 and 1623 in the pharmaceutical unit may be adjusted to achieve a desired drug release profile. Although the drug-containing portions 1622 and 1623 as shown in the figure are single-layer structures, they may be configured as multi-layer structures as shown above.

### Pharmaceutical Dosage Form III

The present application further provides another pharmaceutical dosage form. The pharmaceutical dosage form comprises a retaining member and an unfolding member. The unfolding member comprises at least one elastic portion, the unfolding member being configured such that the unfolding member is in a contracted shape under the actioni of a constraint force, and returns to an unfolding shape when the constraint force is withdrawn. In some embodiments, at least a portion of the elastic portion of the unfolding member is in a bent or folded shape, such as a spring shape, a continuous S-shaped bending, a Z-shaped bending, or an 8-shaped shape, etc., so as to ensure its elastic compression effect.

The retaining member is disposed to surround the unfolding member and applies a constraint force to the unfolding member such that the unfolding member is retained in the contracted shape. In some embodiments, the retaining member may be disposed to be a shell structure that completely coats the unfolding member. In other embodiments, the retaining member may also be a sleeve structure, a strip structure, etc., disposed around only a portion of the unfolding member. The retaining member comprises a water-soluble material and is configured to remove the constraint force from the unfolding member when the water-soluble material is dissolved, thereby allowing the unfolding member to be at least partially unfolded.

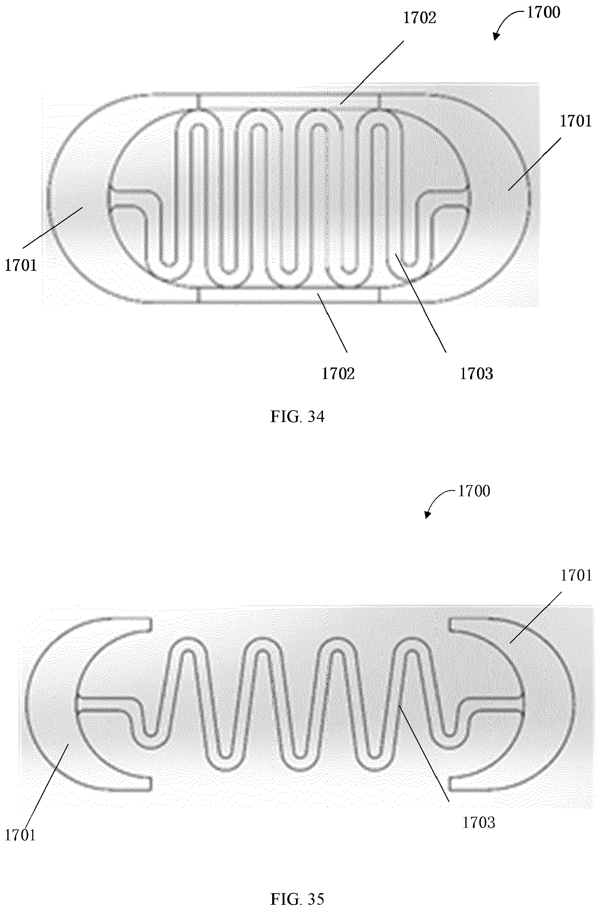

FIGs. 34 and 35 show an example of a pharmaceutical dosage form III. Specifically, FIG. 34 shows a schematic top view of a pharmaceutical dosage form 1700 according to one embodiment of the present application. FIG. 35 shows a schematic top view of the pharmaceutical dosage form 1700 shown in FIG. 34 unfolded with a retaining member removed. As shown in FIGs. 34 and 35, retaining member first portions 1701 and retaining member second portions 1702 of the pharmaceutical dosage form 1700 together form a retaining member arranged around and applying a constraint force to an unfolding member 1703, wherein the retaining member second portions 1702 comprise or are made entirely of a water-soluble material. In some embodiments, the water-soluble material described above may include the listing of water-soluble immediate-release thermoplastic materials used in the retaining member in the Pharmaceutical Dosage Form I section of the present application, which will not be described herein again. The retaining member first portions 1701 are connected to the unfolding member 1703, as shown in FIG. 35. Since the retaining member second portions 1702 are dissolved first, the retaining member first portions 1701 are unfolded along with the unfolding member 1703 and, together with the unfolding member 1703, form a structure which is retained within the stomach or other parts of the upper gastrointestinal tract.

In some embodiments, the retaining member first portions 1701 may comprise a drug, for example, the retaining member 1701 may employ the structure and the material of the "pharmaceutical unit" described in the Pharmaceutical Dosage Form I section, thereby achieving a slow release of the drug within the stomach or other parts of the upper gastrointestinal tract. In other embodiments, the retaining member first portions 1701 may further comprise a portion of an enteric drug or material, thereby enabling a further drug release within the intestinal tract upon subsequent entry thereof into the intestinal tract. In some embodiments, the retaining member 1702 may also comprise a drug, such as some drugs that need to be released first in the stomach or the upper gastrointestinal tract. Of course, the retaining member second portions 1702 may employ a drug-loading structure similar to the pharmaceutical unit described in the Pharmaceutical Dosage Form I section, which will not be described herein again. Although not shown, in some embodiments, the unfolding member 1703 may also comprise a drug, as will be described in detail in the following embodiments.

Although the unfolding member 1703 is shown in FIGs. 34 and 35 as an S-shaped continuously folded shape, in other embodiments, the unfolding member 1703 may be folded in any other shape that can effectively reduce the extension length of the pharmaceutical unit in a certain direction, for example, an 8-shaped or Z-shaped continuously folded shape, etc. In still other embodiments, the unfolding member 1703 may also be strip-shaped when fully unfolded, or be configured as an elongated member in other shapes that facilitate bending or folding, for example, in the shape of a rope, a rod, or a cuboid. As shown in FIGs. 34 and 35, the unfolding member 1703 is configured to extend substantially along a plane. This configuration is obviously more suitable for 3D printing and effectively improves the production efficiency of drugs as compared with a spring or other three-dimensionally arranged elastic configuration.

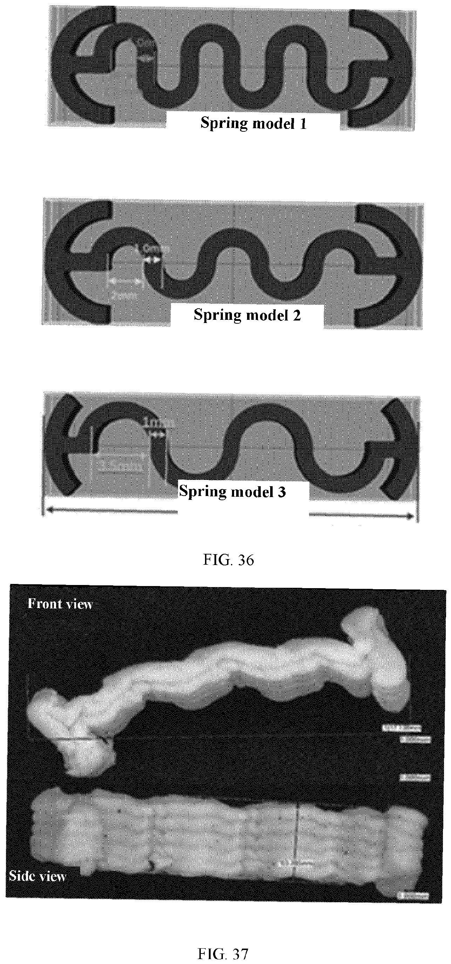

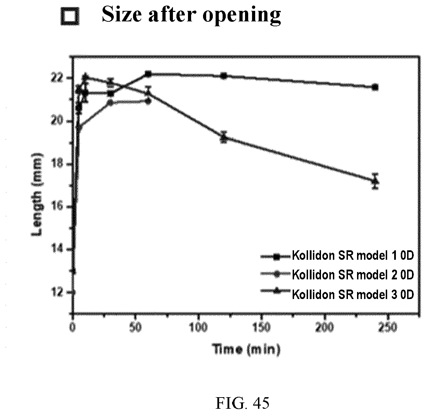

FIG. 36 shows schematic diagrams of structures of three pharmaceutical dosage forms similar to the pharmaceutical dosage form shown in FIG. 34. For the sake of simplicity, the three pharmaceutical dosage form structures in the figure are not shown to have structures corresponding to the retaining member second portions 1702. As shown in the figure, although the unfolding members of the three pharmaceutical dosage form structures are all configured as an S-shaped continuously folded or bent structure similar to the unfolding member 1703, there are differences in the number of times they are folded or bent. In other words, the folding intervals of the bent or folded portions of the unfolding members of the three pharmaceutical dosage form structures are different. Specifically, as shown in the figure, the folding interval of the spring model 1 is 1.5 mm, the folding interval of the spring model 2 is 2 mm, and the folding interval of the spring model 3 is 3.5 mm. Table 1 shows the data of the original length, the compressed length, and the length after opening for one hour In the gastric acid environment for the three pharmaceutical dosage forms shown in FIG. 36, while FIG. 45 shows the corresponding size-time response curves for the three different pharmaceutical dosage forms shown in FIG. 36.

**Table 1. Length of pharmaceutical dosage form**

| | **Original length (mm)** | **Compressed length (mm)** | **Length after opening for 1 h (mm)** |
|---|---|---|---|
| Model 1 (interval: 1.5 mm) | 21.53 | 13.01 | 22.20 |
| Model 2 (interval: 2 mm) | 19.75 | 12.02 | 20.01 |
| Model 3 (interval: 3.5 mm) | 23.09 | 13.00 | 21.18 |

As shown in FIG. 36, the thickness of the unfolding members of all three models is 1 mm. As can be seen from Table 1 and FIG. 45, when the ratio of the folding interval of the unfolding member to the thickness of the unfolding member itself is less than 3:1, the pharmaceutical dosage form can be administered in a smaller size, and after unfolding, it can stably maintain the maximum unfolding size without rapid contraction.

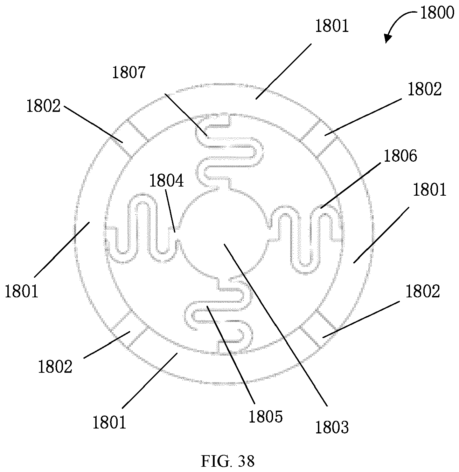

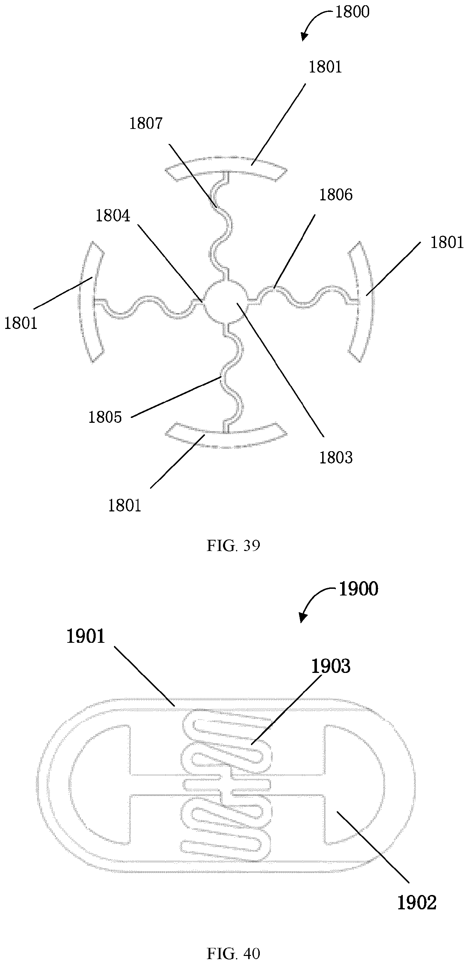

In some embodiments, the unfolding member of the pharmaceutical dosage form III also comprises a first portion and a second portion. FIG. 38 shows a schematic top view of a pharmaceutical dosage form 1800 according to another embodiment of the present application. FIG. 39 shows a schematic top view of the pharmaceutical dosage form 1800 shown in FIG. 38 unfolded with a portion of a retaining member removed. As shown in FIGs. 38 and 39, the pharmaceutical dosage form 1800 comprises retaining member first portions 1801 and retaining member second portions 1802 that together form a retaining member surrounding and applying a constraint force to the unfolding member. The configuration and material for the retaining member first portions 1801 and 1802 are substantially similar to those described in the embodiment of FIG. 34, which will not be described herein again.

With continued reference to FIGs. 38 and 39, the unfolding member of the pharmaceutical dosage form 1800 comprises an unfolding member first portion 1803 and a plurality of portions extending from the unfolding member first portion 1803, namely an unfolding member second portion 1804, third portion 1805, fourth portion 1806, and fifth portion 1807. The unfolding member second portion 1804, third portion 1805, fourth portion 1806, and fifth portion 1807 are connected to the four retaining member first portions 1801, respectively. After the retaining member second portions 1802 are dissolved first and release the constraint on the unfolding member, under the action of elasticity, the unfolding member second portion 1804, third portion 1805, fourth portion 1806, and fifth portion 1807, which are previously in a bent state, extend outward and, together with the four retaining member first portions 1801, form a configuration of a certain unfolding size (e.g., greater than 16 mm), thereby achieving the gastric retention effect as previously described. It should be noted that, although the unfolding member as shown in the figures comprises four other portions of the unfolding member extending from the unfolding member first portion 1803 and connected to the retaining member first portions 1801, in other embodiments, it may also be configured to have 1, 2, 3, 5, 6, or 8 other portions of the unfolding member.

It should be noted that, in some embodiments, the unfolding member first portion 1803 may comprise a water-soluble material and be configured such that when the water-soluble material is dissolved, the unfolding member second portions to fifth portions are separated from each other, thereby effectively reducing the overall size of the pharmaceutical dosage form and allowing it to be expelled smoothly from the stomach. Although not shown in the figures, in some embodiments, it may also be the case that the connection points between the unfolding member first portion 1803 and the unfolding member comprise a water-soluble material, thereby achieving the disassembly effect of the overall structure as described above through the dissolution of the water-soluble material of these connection points. In some embodiments, the dissolution rate in gastric juice of the water-soluble material of the unfolding member first portion 1803 or the water-soluble material of the connection points thereof with other portions described above is configured to be less than that of the retaining member second portions 1802, such that the pharmaceutical dosage form can be smoothly unfolded into the configuration shown in FIG. 39 that enables gastric retention, and can be effectively decomposed and expelled from the stomach after a certain period of gastric retention has been completed.

In some embodiments, the retaining member first portions 1801, the retaining member second portions 1802, or the unfolding member first portion 1803 may all comprise the structure and the material of the "pharmaceutical unit" as described above in the Pharmaceutical Dosage Form I section, so as to achieve drug loading and release. In other embodiments, the second to fifth portions extending from the unfolding member first portion may also employ similar drug loading result. In some embodiments, the unfolding member second to fifth portions may comprise a hydrophobic material and a enteric material to achieve a gastric retention effect.

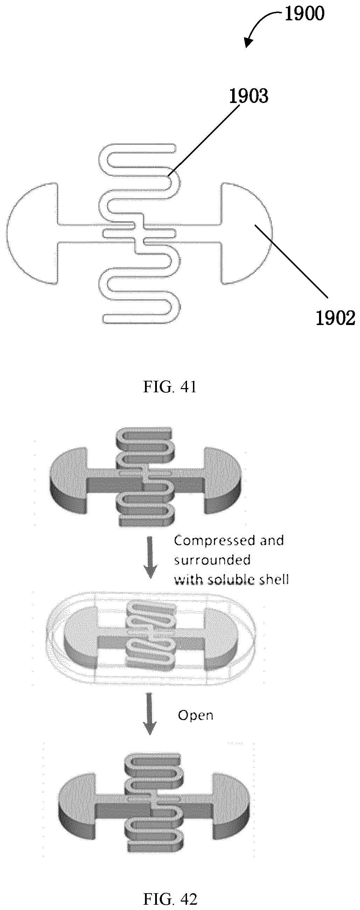

FIG. 40 shows a schematic top view of a pharmaceutical dosage form 1900 according to one embodiment of the present application. FIG. 41 shows a schematic top view of the pharmaceutical dosage form 1900 shown in FIG. 40 unfolded with a retaining member removed. As shown in FIGs. 40 and 41, the pharmaceutical dosage form 1900 comprises a retaining member 1901, an unfolding member first portion 1902, and an unfolding member second portion 1903, with the retaining member 1901 being a shell structure encasing the unfolding member first portion 1902 and the unfolding member second portion 1903. Within the shell formed by the retaining member 1901, the unfolding member first portion 1902 and the unfolding member second portion 1903 are arranged in a cross-connection with each other, such that when the retaining member 1901 is dissolved at least partially and releases the constraints on the unfolding member first portion 1902 and the unfolding member second portion 1903, due to the cross arrangement of the two, the gastric retention effect can be better achieved.

The unfolding member first portion 1902 may be configured to comprise the drug-loading structure and the material of the "pharmaceutical unit" described in the Pharmaceutical Dosage Form I section. For example, in some embodiments, both ends of the unfolding member first portion 1902 each comprise at least one compartment, each of which independently comprises the same or a different drug therein. In some embodiments, the unfolding member second portion 1903 also comprises a drug therein. In other embodiments, the retaining member 1901 may also comprise a drug therein that needs to be released in advance or rapidly in the stomach, so as to achieve a release of the entire drug in batches and under different conditions for optimal therapeutic effect. In some embodiments, the connection points between the unfolding member first portion 1902 and the unfolding member second portion 1903 comprise a water-soluble material, such that through the dissolution of the water-soluble material of these connection points, separation of the unfolding member first portion 1902 from the unfolding member second portion 1903 after a certain time is achieved to facilitate their expulsion from the stomach.

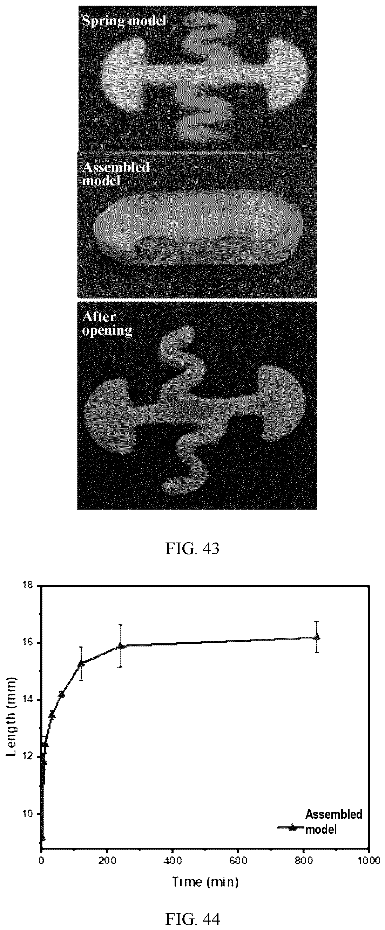

FIG. 42 shows a 3D modeling of the pharmaceutical dosage form 1900 shown in FIG. 40. FIG. 43 shows the physical pictures of an unconstrained unfolding member, a printed pharmaceutical dosage form, and an unfolded pharmaceutical dosage form of the pharmaceutical dosage form 1900 shown in FIG. 40.

It should be noted that the water-soluble material mentioned in the above embodiments of the pharmaceutical dosage form III may include the listing of water-soluble immediate-release thermoplastic materials used in the retaining member in the Pharmaceutical Dosage Form I section of the present application, which will not be described herein again. In addition, the material used in the elastic portion of the unfolding member can refer to the listing of elastomer materials defined in the Pharmaceutical Dosage Form I section, and the material contained in the "pharmaceutical unit" can be the specific material and option in the description of the pharmaceutical unit in the Pharmaceutical dosage form I section, which will not be described herein again. In some embodiments, the pharmaceutical dosage form may be formed by printing with a 3D printing method. In some embodiments, at least a portion of the unfolding member or the retaining member of the pharmaceutical dosage form described above further comprises a swellable material such that it can swell in gastric juice to match the unfolding of the unfolding member, thereby achieving a better gastric retention effect and reducing the size of the product.

### Method for three-dimensional printing of oral pharmaceutical dosage form

The present application provides a method for preparing the pharmaceutical dosage form I described herein by three-dimensional printing. In some embodiments, the method specifically comprises: printing a pharmaceutical unit containing a drug; printing at least one unfolding member such that the unfolding member is connected to the pharmaceutical unit, wherein the unfolding member comprises at least one elastic portion configured such that the unfolding member is in a contracted shape proximal to the pharmaceutical unit under the action of a constraint force, and returns to an unfolding shape distal to the pharmaceutical unit when the constraint force is withdrawn; and printing a retaining member such that the retaining member is connected to the at least one unfolding member and applies a constraint force to the at least one unfolding member to cause the unfolding member to be in the contracted shape; wherein the retaining member comprises a water-soluble material and the retaining member is configured to remove the constraint force from the unfolding member when the water-soluble material is dissolved, such that the at least one unfolding member can change from the contracted shape to the unfolding shape.

In some embodiments, the step of printing the retaining member specifically comprises: printing a retaining member such that the retaining member at least partially encases the pharmaceutical unit and the at least one unfolding member, such that the at least one unfolding member is in a contracted shape. It should be noted that in some embodiments, the unfolding member of the printed pharmaceutical dosage form may also not comprise an elastic portion, and is configured to require an external force to move from the contracted shape to the unfolding shape. Specifically, in some embodiments, the unfolding member portion of the pharmaceutical dosage form may comprise a portion with reduced thickness, which has a thickness set to be less than that of the adjacent portion. Since the portion with reduced thickness is relatively easy to bend, the unfolding member still easily moves from the contracted shape to the unfolding shape under the action of gastric juice or other substances. By using the pharmaceutical dosage form constructed with the portion with reduced thickness, a similar unfolding effect can be achieved without particularly employing an elastic material, thereby effectively reducing costs. In addition, since there is no need to employ an elastic material, when the pharmaceutical dosage form is printed by using a 3D printing technique, its unfolding member can be printed with the same material as a whole, avoiding multiple switching of the printing head. Meanwhile, the pharmaceutical dosage form can also be printed directly in a contracted shape, which effectively improves the production efficiency of the final product.

In some embodiments, the step of printing the retaining member specifically comprises: printing a retaining member such that the retaining member is arranged between the pharmaceutical unit and the unfolding member, and is connected to the pharmaceutical unit, such that the unfolding member is in a contracted shape. In other embodiments, the pharmaceutical dosage form to be printed comprises a plurality of unfolding members, and the step of printing the retaining member specifically comprises: printing a retaining member such that at least two unfolding members are connected by the retaining member, thereby constraining the two unfolding members in a contracted state. In some embodiments, the pharmaceutical unit of the pharmaceutical dosage form comprises a drug-containing portion and a drug-free portion, the drug-containing portion being printed after the step of printing the retaining member is completed.

The present application further provides a method for preparing a pharmaceutical dosage form II by using a three-dimensional printing process. The method comprises: printing a pharmaceutical unit containing a drug and having at least a portion of the pharmaceutical unit in a bent or folded shape; and printing a retaining member such that the retaining member is connected to the pharmaceutical unit and applies a constraint force to the pharmaceutical unit such that at least a portion of the pharmaceutical unit is retained in the bent or folded shape; wherein the retaining member comprises a water-soluble material and the retaining member is configured to remove the constraint force from the pharmaceutical unit when the water-soluble material is dissolved, thereby allowing at least a portion of the pharmaceutical unit to unfold from the bent or folded shape.

The present application further provides a method for preparing a pharmaceutical dosage form III by using a three-dimensional printing process. The method comprises: printing an unfolding member comprising at least one elastic portion configured such that the unfolding member is in a contracted shape under a constraint force, and returns to an unfolding shape when the constraint force is withdrawn; applying a constraint force to the unfolding member such that the unfolding member is retained in the contracted shape; and printing a retaining member to retain the constraint force on the unfolding member by the retaining member such that the unfolding member is retained in the contracted shape; wherein the retaining member comprises a water-soluble material and the retaining member is configured to remove the constraint force from the unfolding member when the water-soluble material is dissolved, thereby allowing the unfolding member to be at least partially unfolded.

In some embodiments, the step of printing the retaining member specifically comprises: printing an unfolding member first, applying a constraint force to the printed unfolding member through a clamp to enable the unfolding member to be in a contracted shape, and when the unfolding member is in a contracted state, printing a first portion of the retaining member connected to the unfolding member first and then printing a second portion of the retaining member. In some embodiments, the step of printing the retaining member specifically comprises: printing a retaining member first, printing an unfolding member in a contracted shape under a constraint force by the retaining member in a range surrounded by the retaining member such that the unfolding member is connected to the retaining member, wherein the unfolding member comprises at least one elastic portion configured such that the unfolding member is at least partially unfolded when the constraint force applied by the retaining member is removed, and the 3D printed unfolding member is subjected to a heat treatment to increase the elasticity of the unfolding member. It should be noted that, as used herein, "printing", "three-dimensional printing", "3D printing", "additive manufacturing", or synonyms thereof refer to a process for producing a three-dimensional object (e.g., a pharmaceutical dosage form) by layer-by-layer stacking using a digital design. The basic process of 3D printing is described in U.S. Patent Nos. US5204055, US5260009, US5340656, US5387380, US5503785, and US5633021. In addition, other U.S. patents and patent applications related to 3D printing include: U.S. Patent Nos. US5490962, US5518690, US5869170, US6530958, US6280771, US6514518, US6471992, and US8828411, and U.S. Patent Application Nos. US2002/0015728, US2002/0106412, US2003/0143268, US2003/0198677, and US2004/0005360. The above U.S. patents and patent applications are incorporated herein by reference in their entirety.

Some articles also disclose 3D printing methods for manufacturing a pharmaceutical dosage form, which are optimized in terms of raw materials, equipment, and curing. For example, these methods include binder deposition (see Gibson et al., "Additive Manufacturing Technologies: 3D Printing, Rapid Prototyping, and Direct Digital Manufacturing", 2nd Edition, Springer, New York, 2015; Katstra et al., "Oral Dosage Forms Fabricated by Three Dimensional Printing", J Control Release, 66, 2000; Katstra et al., Fabrication of Complex Oral Drug Delivery Forms by Three Dimensional Printing, Dissertation in Materials Science and Engineering, Massachusetts Institute of Technology, 2001; Lipson et al., Fabricated: The New World of 3D printing, John Wiley & Sons, Inc., 2013; Jonathan, Karim, 3D printing in pharmaceutics: a new tool for designing customized drug delivery systems, Int J Pharm, 499, 2016), material jetting (see Jonathan, Karim, pharmaceutical field 3D printing: New tools for designing custom drug delivery systems, Int J Pharm, 499, 2016), extrusion (see Gibson et al., "Additive Manufacturing Technologies: 3D Printing, Rapid Prototyping, and Direct Digital Manufacturing", 2nd Edition, Springer, New York, 2015), and photopolymerization (see Melchels et al., A review on stereolithography and its application in bio-medical engineering, Biomaterials, 31, 2010).

In some embodiments, the pharmaceutical dosage forms disclosed herein are 3D printed using an extrusion method. In some embodiments, the method for 3D printing comprises the use of twin-screw extrusion method. In the extrusion process, a material is extruded from a mechanically driven printing head through a printing nozzle. Unlike binder deposition, which requires a powder bed, the extrusion method can print on any substrate. A variety of materials can be extruded for three-dimensional printing, including the thermoplastic materials disclosed herein, pastes and colloidal suspensions, silicones, and other semisolids. One common type of extrusion printing is fused deposition modeling, which uses solid polymer filaments for printing. In fused deposition modeling, a gear system drives the filament into a heated nozzle assembly for extrusion (see Gibson et al., "Additive Manufacturing Technologies: 3D Printing, Rapid Prototyping, and Direct Digital Manufacturing", 2nd Edition, Springer, New York, 2015).

In some embodiments, the 3D printing methods described herein comprise a continuous feed method.

In some embodiments, the 3D printing methods described herein comprise a batch feed method.

In some embodiments, the 3D printing is performed by fused deposition modeling (FDM). In some embodiments, the 3D printing is performed by no-filament FDM. In some embodiments, the 3D printing is performed by melt extrusion deposition (MED). In some embodiments, the 3D printing is performed by inkjet printing. In some embodiments, the 3D printing is performed by selective laser sintering (SLS). In some embodiments, the 3D printing is performed by stereolithography (SLA or SL). In some embodiments, the 3D Printing is performed by Poly Jet, Multi-Jet Printing System (MJP), Perfactory, Solid Object Ultraviolet-Laser Printer, Bioplotter, 3D Bioprinting, Rapid Freeze Prototyping, Benchtop System, Selective Deposition Lamination (SDL), Laminated Objet Manufacutring (LOM), Ultrasonic Consolidation, ColorJet Printing (CJP), EOSINT Systems, Laser Engineered Net Shaping (LENS) and Aerosol Jet System, Electron Beam Melting (EBM), Laser CUSING^{®}, Selective Laser Melting (SLM), Phenix PXTM Series, Microsintering, Digital Part Materialization (DPM), or VX System.

In some embodiments, the three-dimensional printing is performed by hot melt extrusion coupled with a 3D printing technique, such as FDM. In some embodiments, the three-dimensional printing is performed by melt extrusion deposition (MED).

In some embodiments, the method for preparing the oral pharmaceutical dosage form described herein comprises a 3D printing technique, such as a combination of 3D printing and another method, e.g., a combination of injection molding and 3D printing. In some embodiments, the shell is manufactured using injection molding, and the one or more portions are manufactured using a 3D printing technique.

The method for 3D printing a pharmaceutical dosage form disclosed herein may generate instructions in a variety of ways, including direct coding, derivation from a solid CAD model, or other means specific to the computer interface and application software of a 3D printer. These instructions may include information on the number and spatial placement of droplets, and on general 3D printing parameters, such as the droplet spacing in each linear dimension (X, Y, Z) and the volume or mass of liquid per droplet. For a given set of materials, these parameters may be adjusted to refine the quality of the created structure. The overall resolution of the created structure is a function of the powder particle size, the fluid droplet size, the printing parameters, and the material properties.

In some embodiments, the method comprises creating a three-dimensional map of an oral pharmaceutical dosage form based on predetermined parameters of the oral pharmaceutical dosage form, wherein the three-dimensional map is created on a computer system. In some embodiments, the method comprises converting (e.g., slicing) the three-dimensional map of the oral pharmaceutical dosage form into a three-dimensional printing code, e.g., a G-code. In some embodiments, the method comprises executing the three-dimensional printing code using a computer system, thereby printing the oral pharmaceutical dosage form described herein.

### Examples

Specific examples of printing a pharmaceutical dosage form using a three-dimensional printing method will be described below with reference to the drawings in the specification.

### Example 1

Specifically, a method for printing a pharmaceutical dosage form 400 using three-dimensional printing is described in detail with reference to FIGs. 7 to 8.

First, a pharmaceutical unit 402 and unfolding members 401 in an unfolding shape of the pharmaceutical dosage form 400 as shown in FIG. 7 were printed. Although the pharmaceutical unit 402 is shown as a cube in the figure, it will be understood by those skilled in the art that the pharmaceutical unit 402 may also be in the form of a cylinder, a capsule, or other orally acceptable drug forms, which has a shell portion and a drug-containing inner core, wherein the shell has a "U" shaped profile, and the drug inner core is loaded in a compartment formed by the outer shell. In addition, the shell portion was made of the same material as the unfolding members 401. The material of the outer shell of the pharmaceutical unit 402 was composed of quaternary amino methacrylate copolymer type B and ethylcellulose; the drug-containing inner core was composed of model drug and hydroxypropyl cellulose; and the material of the retaining member was polyvinylpyrrolidone Kollidon VA64. Specifically, the material of the outer shell (same material as the unfolding members 401), the material of the drug-containing inner core, and the material of the retaining member of the pharmaceutical unit 402 were separately placed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted material of the outer shell (same material as the unfolding members 401), material of the drug-containing inner core, and material of the retaining member were fed into a printing head 1, a printing head 2, and a printing head 3, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, a G-code set by a three-dimensional slice of a drug model was imported, and the pharmaceutical unit and the unfolding members 401 in the unfolding shape began to be printed layer by layer, as shown in FIG. 7. Each layer was printed layer by layer by switching between the printing head 1 and the printing head 2, wherein the unfolfding members 401 were connected to the bottom of the pharmaceutical unit 402, and the connection portions were designed as thinner connections through the drug model.

After the printing of the pharmaceutical unit 402 and the unfolding members 401 was completed, the unfolding members 401 were folded upwards using a gripper, a mechanical arm, or other mechanical workpieces to encase the pharmaceutical dosage form 400 in the form shown in FIG. 8. Although the 401 is shown to fully encase the upper surface of the pharmaceutical unit 402 in the figure, it will be understood by those skilled in the art that by setting the length of the unfolding members 401, it is also possible to encase only the outer peripheral sidewalls of the pharmaceutical unit 402, leaving the drug inner core on the upper surface of the pharmaceutical unit 402 exposed.

Next, the printing head 3 loaded with the melted material of the retaining member continued to print a seal on the upper portion of the pharmaceutical dosage form 400 in a contracted shape, such that a coating of the retaining member was printed to completely or partially encase the outer surface of the pharmaceutical dosage form 400 in the contracted shape. The retaining member of the pharmaceutical dosage form 400 may also encase the positions where the different unfolding members 401 meet or are close to each other after folding.

### Example 2

Specifically, a method for printing a pharmaceutical dosage form 700 using three-dimensional printing is described in detail with reference to the structure of the pharmaceutical dosage form 700 shown in FIGs. 11 to 12.

First, a pharmaceutical dosage form as shown in FIG. 11 was printed, comprising a pharmaceutical unit 702, elastic portions 711, and unfolding portions 701. The pharmaceutical unit 702 comprised a shell portion, a drug-containing inner core, and a plug, and the unfolding members 701 and the shell portion of the pharmaceutical unit 702 were made of the same material.

The pharmaceutical unit was in the shape of a cylinder, and the outer shell had a "U" shaped profile. The drug inner core was loaded in a compartment formed by the outer shell, which was sealed with the plug. The material of the outer shell of the pharmaceutical unit 702 was composed of quaternary amino methacrylate copolymer type B and ethylcellulose; the drug-containing inner core was composed of model drug and hydroxypropyl cellulose; and the plug was composed of hydroxypropyl cellulose and triethyl citrate. The material of the elastic portions 711 was a styrene block copolymer. The material of the retaining member was polyvinylpyrrolidone Kollidon VA64. The material of the outer shell (same material as the unfolding member), the material of the drug-containing inner core, the material of the plug, the material of the elastic portions, and the material of the retaining member of the pharmaceutical unit were separately fed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted material of the outer shell (same material as the unfolding member), material of the drug-containing inner core, material of the plug, material of the elastic portions, and material of the retaining member were fed into a printing head 1, a printing head 2, a printing head 3, a printing head 4, and a printing head 5, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, a G-code set by a three-dimensional slice of a drug model was imported, and an unfolding structure of the pharmaceutical dosage form began to be printed layer by layer, as shown in FIG. 11. Each layer was selectively printed layer by layer by switching between the printing head 1, the printing head 2, the printing head 3, and the printing head 4.

After the printing of the unfolding structure was completed, the two unfolding members 701 were moved to a contracted shape with the distal ends adjacent to each other using a gripper, a mechanical arm, or other mechanical workpieces, as shown in FIG. 12.

Subsequently, by using a 3D printing head, a retaining member 731 was printed between the distal ends of the two unfolding members 701 to connect the distal ends of the two, and retaining members 732 were printed between the pharmaceutical unit 702 and the unfolding members 701. It should be noted that the retaining member 731 in Example 3 may either be printed layer by layer in the vertical direction using a 3D printing head to achieve the connection between the unfolding members 701, or be printed in the vertical direction using a five-axis 3D printing head.

### Example 3

Specifically, a method for printing a pharmaceutical dosage form 800 using three-dimensional printing is described in detail with reference to FIGs. 13 to 16.

First, a pharmaceutical unit 802 and unfolding members 801 in an unfolding shape of the pharmaceutical dosage form 800 as shown in FIGs. 13 and 14 were printed. The pharmaceutical unit 802 had a shell portion and a drug-containing inner core, and the shell portion was made of the same material as the unfolding members 801. The material of the outer shell of the pharmaceutical unit 802 was composed of quaternary amino methacrylate copolymer type B and ethylcellulose; the drug-containing inner core was composed of model drug and hydroxypropyl cellulose; and the material of the retaining member 801 was polyvinylpyrrolidone Kollidon VA64. Specifically, the material of the outer shell (same material as the unfolding members), the material of the drug-containing inner core, and the material of the retaining member of the pharmaceutical unit 802 were separately placed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted material of the outer shell (same material as the unfolding members), material of the drug-containing inner core, and material of the retaining member were fed into a printing head 1, a printing head 2, and a printing head 3, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, a G-code set by the three-dimensional slice of the drug model was imported, and the pharmaceutical unit and the unfolding members 801 in the unfolding shape began to be printed layer by layer, as shown in FIG. 13. Each layer was printed layer by layer by switching between the printing head 1 and the printing head 2, wherein the connection portions of the unfolding members 801 and the pharmaceutical unit 802 were designed as thinner connections through the drug model.

After the printing of the unfolding members 801 was completed, the unfolding members 801 were folded upwards using a gripper, a mechanical arm, or other mechanical workpieces to encase portions of the outer peripheral sidewalls of the pharmaceutical unit 802.

Next, the printing head 3 loaded with the melted material of the retaining member continued to print on the upper portions of the pharmaceutical unit 802 and the unfolding members 801, such that the structure of the retaining member 803 as shown in FIGs. 15 and 16 was printed to encase the remaining portions of the outer peripheral sidewalls of the pharmaceutical unit 802, as well as the upper surfaces of the unfolding members 803 in the contracted shape and the pharmaceutical unit 802.

The pharmaceutical dosage form 800 may also comprise elastic portions that connect the unfolding members 801 and the pharmaceutical unit 802. After the pharmaceutical dosage form 800, which is constrained into the contracted shape, is administered to a human body, along with the rapid erosion of the retaining member 803, the elastic portions or materials enable the unfolding members of the pharmaceutical dosage form to automatically return from the contracted shape to the unfolding shape. For printing of a pharmaceutical dosage form having elastic portions, it will be understood by those skilled in the art that the printing of the pharmaceutical unit 802, the unfolding members 801, and the elastic portions connecting the two can be achieved by adding a printing head 4 to load a melted elastic material and printing layer by layer by switching between the printing heads 1, 2, and 4, and then the unfolding members 801 are folded upwards by a gripper, a mechanical arm, or other mechanical workpieces to encase portions of the outer peripheral walls of the pharmaceutical unit 902, leaving the elastic portions in a deformed state. Finally, the pharmaceutical dosage form is sealed, encased, or adhered by the printing head 3 loaded with the melted material of the retaining member, and a tablet with a size easy for a patient to take orally is manufactured.

### Example 4

Specifically, a method for printing a pharmaceutical dosage form 900 using three-dimensional printing is described in detail with reference to the structure of the pharmaceutical dosage form 900 shown in FIGs. 17 to 20.

First, a pharmaceutical unit 902, an unfolding member 901, and a retaining member 903 as shown in FIGs. 17 and 19 were printed, wherein the pharmaceutical unit 902 comprised a shell portion and a drug-containing inner core, and the unfolding member 901 and the shell portion of the pharmaceutical unit 902 were made of the same material. The material of the outer shell of the pharmaceutical unit was composed of quaternary amino methacrylate copolymer type B and ethylcellulose; the drug-containing inner core was composed of model drug and hydroxypropyl cellulose; and the material of the retaining member was polyvinylpyrrolidone Kollidon VA64. The material of the outer shell (same material as the unfolding members), the material of the drug-containing inner core, and the material of the retaining member of the pharmaceutical unit were separately fed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted material of the outer shell (same material as the unfolding members), material of the drug-containing inner core, and material of the retaining member were fed into a printing head 1, a printing head 2, and a printing head 3, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, a G-code set by a three-dimensional slice of a drug model was imported, and an unfolding structure of the pharmaceutical dosage form began to be printed layer by layer, as shown in FIG. 17. Each layer was printed layer by layer by switching between the printing head 1 and the printing head 2, wherein the connection portion of the unfolding member 901 and the pharmaceutical unit 902 was designed as a thinner connection through the drug model.

After the printing of the unfolding structure was completed, the unfolding member 901 was wound around the pharmaceutical unit 902 to form a contracted shape as shown in FIG. 18 using a gripper, a mechanical arm, or other mechanical workpieces.

Next, the printing head 3 loaded with the melted material of the retaining member continued to print on the upper portion of the pharmaceutical unit 902, such that the structure of the retaining member 903 as shown in FIGs. 19 and 20 was printed to encase the upper half of the outer peripheral sidewall of the pharmaceutical unit 902, as well as the upper and outer peripheral side parts of the unfolding member 903 in the contracted shape.

The pharmaceutical dosage form may also comprise an elastic portion that connects the unfolding member 901 and the pharmaceutical unit 902. The elastic portion or material enables the pharmaceutical dosage form to be rolled or folded from the unfolding shape to the contracted shape during the printing process and allows the pharmaceutical dosage form to return from the contracted shape to the unfolding shape when administered to a human body. It will be understood by those skilled in the art that the unfolding structure of the pharmaceutical dosage form can be achieved by adding a printing head 4 to load a melted elastic material and printing layer by layer by switching between the printing heads 1, 2, and 4, and then the elastic member and the unfolding member 901 are deformed and wound around the pharmaceutical unit 902 to form a contracted shape as shown in FIG. 18 by a gripper, a mechanical arm, or other mechanical workpieces. Finally, the pharmaceutical dosage form is sealed, encased, or adhered by the printing head 3 loaded with the melted material of the retaining member, and a tablet with a size easy for a patient to take orally is manufactured.

The order of the printing steps in Example 1 described above can also be adapted arbitrarily. In addition, the unfolding member 901 in the contracted shape may also be directly printed in a mold without the step of moving the unfolding member in the unfolding shape to the contracted shape.

### Example 5

As previously described, the unfolding member 901 may also return from the contracted shape to the unfolding shape without relying on an elastic portion or material. For example, the unfolding member 901 may comprise a portion with reduced thickness that is relatively easy to bend, such that the unfolding member still easily unfolds from the contracted shape to the unfolding shape under the action of gastric juice or other substances.

For a pharmaceutical dosage form constructed with an unfolding member having a portion with reduced thickness, it preferably comprises a plurality of unfolding members, for example, 3, 4, 5, 6 unfolding members 101 as shown in FIG. 1. These unfolding members are pivotable relative to their respective portions with reduced thickness under the action of gastric juice or other substances. Since the plurality of unfolding members located at different positions on the outer peripheral wall of the pharmaceutical unit rarely pivot to a contracted state at the same time, the pharmaceutical dosage form can still maintain a relatively unfolded shape in gastric juice to ensure the retention effect.

For a pharmaceutical dosage form in which the structure of the pharmaceutical unit and the retaining member is similar to that of Example 1 but a plurality of unfolding members as shown in FIG. 1 are employed, the corresponding overall printing method is the same as in Example 1, except that the steps of printing the shell and the unfolding member are adapted to print a shell and an unfolding member in a contracted shape, so as to omit the subsequent step of moving the unfolding member to the contracted shape. It should be emphasized here that printing the shell and the unfolding member in the contracted shape specifically comprises printing the shell and the unfolding member such that the unfolding member is adjacent to the shell but maintains a certain gap to avoid adhesion between the two.

### Example 6

Specifically, a method for printing a pharmaceutical dosage form 1200 using three-dimensional printing is described in detail with reference to FIGs. 22 to 26.

First, a pharmaceutical dosage form as shown in FIG. 26 was printed, comprising a pharmaceutical unit 1204, a floating member 1207, unfolding members 1201, and a retaining member 1203. The pharmaceutical unit 1204 comprised a shell portion, drug-containing inner cores, and plugs 1205, with the drug-containing inner cores loaded in compartments 1206, and the unfolding members 1201, the floating member 1207, and the shell portion of the pharmaceutical unit 1204 were made of the same material.

The pharmaceutical unit 1204 was enclosed by the outer shell to form a plurality of compartments, and the drug inner cores were loaded in the compartments 1206 formed by the outer shell, and sealed by the plugs 1205 containing no drug. The material of the outer shell of the pharmaceutical unit 1204 was composed of quaternary amino methacrylate copolymer type B and ethylcellulose; the drug-containing inner cores were composed of model drug and hydroxypropyl cellulose; and the plugs were composed of hydroxypropyl cellulose and triethyl citrate. The material of the retaining member was polyvinylpyrrolidone Kollidon VA64. The material of the outer shell (same material as the unfolding members and the floating member), the material of the drug-containing inner cores, the material of the plugs, and the material of the retaining member of the pharmaceutical unit were separately fed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted material of the outer shell (same material as the unfolding members and the floating member), material of the drug-containing inner cores, material of the plugs, and material of the retaining member were fed into a printing head 1, a printing head 2, a printing head 3, and a printing head 4, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, and the filling rate of the floating member was set to 10%. A G-code set by a three-dimensional slice of a drug model was imported, and an unfolding structure of the pharmaceutical dosage form began to be printed layer by layer. First, each layer was selectively printed layer by layer by the printing head 1 to form a floating member 1207 with a grid structure and unfolding members 1201.

Subsequently, the unfolding members 1201 in the unfolding shape were moved to a contracted shape as shown in FIG. 26 using a gripper, a mechanical arm, or other mechanical workpieces. Next, a retaining member 1203 was printed using the printing head 4 loaded with the melted material of the retaining member, such that the printed portion encased the outer peripheral wall of the floating member 1207 and the upper portions of the unfolding members 1201.

After the printing of the floating member 1207, the unfolding members 1201, and the retaining member 1203 was completed, the pharmaceutical unit 1204 was printed. Specifically, the outer shell, the drug-containing inner cores loaded in the compartments 1206, and the plugs 1205 as shown in FIG. 26 can be printed in layers using the printing head 1, the printing head 2, and the printing head 3 alternately.

It should be noted that, since the step of printing the drug-containing portion can be performed independently of other structures of the pharmaceutical dosage form, the above printing method can employ any other design, method and procedure for printing a 3D pharmaceutical dosage form, thereby rapidly printing the gastroretentive dosage form of the drug release structure. In some embodiments, the drug-containing portion may be printed first, followed by other portions of the pharmaceutical dosage form.

### Example 7

A method for printing a pharmaceutical dosage form 1500 using three-dimensional printing is described in detail below with reference to FIGs. 30 and 31.

The pharmaceutical unit 1502 comprised a material of an outer shell 1521, a drug-containing layer 1522, and a retaining member 1503. The material of the outer shell was composed of one or more of hydrophobic materials such as ethylcellulose, polyethylene, beeswax, Eudragit RS, and Eudragit RL; the drug-containing inner core was composed of a drug model and one or more of polyvinylpyrrolidone Kollidon VA64, Soluplus, polyethylene glycol (PEG200, PEG300, PEG400, PEG600, PEG1000, and PEG1500) and polymethyl acrylate (Eudragit E, L100, L100-55, and S100); and the material of the retaining member was polyvinylpyrrolidone Kollidon VA64.

The material of the outer shell, the material of the drug-containing inner core, and the material of the retaining member of the pharmaceutical unit were separately fed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted material of the outer shell (same material as the unfolding members), material of the drug-containing inner core, and material of the retaining member were fed into a printing head 1, a printing head 2, and a printing head 3, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, a G-code set by a three-dimensional slice of a drug model was imported, and an unfolding structure of the pharmaceutical dosage form began to be printed layer by layer, as shown in FIG. 30. Each layer was printed layer by layer by switching between the printing head 1, the printing head 2, and the printing head 3. Specifically, a pharmaceutical dosage form structure in which the pharmaceutical unit was in a helical shape and the retaining member filled the gap of the helical shape as shown in FIG. 30 was printed in layers using the above three 3D printing heads alternately.

Since the above method does not involve a step of mechanically moving the pharmaceutical unit to allow it to be in a contracted shape, the product manufacturing efficiency can be effectively improved, and the stability of the contracted shape configuration of the pharmaceutical unit after printing can be ensured. In addition, the above method also facilitates the designer to adjust the bending shape of the pharmaceutical unit as desired, such as S-shaped, Z-shaped folding, etc., as described above, thus adapting to different usage requirements. Of course, it is also contemplated to print different portions of the pharmaceutical dosage form 1500 separately, for example, the pharmaceutical unit 1502 in the helical shape is printed first, followed by the retaining member 1503 to fill the gap therebetween.

The pharmaceutical dosage form printed by the three-dimensional printing method as described in Example 5 has both a hydrophilic surface (the drug-containing portion 1522) and a hydrophobic surface (the shell portion 1521), such that it can unfold from an at least partially bent shape under the action of surface tension after the rapid dissolution of the retaining member 1503.

### Example 8

Specifically, a method for printing a pharmaceutical dosage form 1700 using three-dimensional printing is described in detail with reference to the structure of the pharmaceutical dosage form 1700 shown in FIGs. 34 to 35.

First, a pharmaceutical dosage form as shown in FIG. 34 was printed, comprising retaining member first portions 1701, retaining member second portions 1702, and an unfolding member 1703.

The unfolding member 1703 was made of povidone Kollidon SR and was in an S-shaped continuously folded shape. The retaining member first portions 1701 were in the shape of horseshoe-shaped columns, having compartments therein in which a drug was loaded. The retaining member first portions 1701 were made of a material including, but not limited to, erodible materials as defined in the Pharmaceutical Dosage Form I section. The retaining member second portions 1702 and the retaining member first portions 1701 were connected and together formed an elliptical cylinder, enclosing the unfolding member 1703 in a contracted state. The retaining member second portions 1702 comprised, but was not limited to, a water-soluble material as defined in the Pharmaceutical Dosage Form I section. The material of the unfolding member, the material of the retaining member first portions, and the material of the retaining member second portions were separately fed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted material of the unfolding member, material of the retaining member first portions, and material of the retaining member second portions were fed into a printing head 1, a printing head 2, and a printing head 3, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, a G-code set by a three-dimensional slice of a drug model was imported, and an unfolding structure of the pharmaceutical dosage form began to be printed layer by layer, as shown in FIG. 35.

After the printing of the unfolding member was completed, the retaining member first portions were printed, and the unfolding member and the retaining member first portions were moved to a contracted shape with the distal ends adjacent to each other using a gripper, a mechanical arm, or other mechanical workpieces, as shown in FIG. 34.

Subsequently, by using a 3D printing head, retaining member second portions 1702 were printed between the distal ends of the retaining member first portions 1701 to connect the distal ends of the two.

The printing process of Example 8 may also be performed in the following order:

Retaining member first portions 1701 and second portions 1702 are printed first, and an unfolding member 1703, which is under a constraint force by the retaining member, is printed within a range surrounded by the retaining member, such that the unfolding member 1703 is connected to the retaining member first portions 1701. After the printing was completed, the unfolding member was subjected to a heat treatment to increase the elasticity of an elastic portion.

It should be noted that retaining member second portions 1702 in Example 8 may either be printed layer by layer in the vertical direction using a 3D printing head to achieve the connection between the retaining member first portions 1701, or be printed in the vertical direction using a five-axis 3D printing head.

### Example 9

Specifically, a method for printing a pharmaceutical dosage form 1800 using three-dimensional printing is described in detail with reference to the structure of the pharmaceutical dosage form 1800 shown in FIGs. 38 to 39.

First, a pharmaceutical dosage form as shown in FIG. 38 was printed, comprising retaining member first portions 1801, retaining member second portions 1802, an unfolding member first portion 1803, an unfolding member second portion 1804, and an unfolding member third portion 1805.

The unfolding member first portion 1803 and the unfolding member second portion 1804 were made of a material including, but not limited to, elastomeric materials as defined in the Pharmaceutical Dosage Form I section, and were in an S-shaped continuously folded shape. The unfolding member first portion 1803 and the unfolding member second portion 1804 were cross-connected to each other through the unfolding member third portion 1805. The unfolding member third portion 1805 was made of a material including, but not limited to, erodible materials as defined in the Pharmaceutical Dosage Form I section, having a compartment therein in which a drug was loaded. The retaining member first portions 1801 and the retaining member second portions 1802 were in the shape of a circular arc, having compartments therein in which a drug was loaded. The retaining member first portions 1801 were made of a material including, but not limited to, erodible materials as defined in the Pharmaceutical Dosage Form I section. The retaining member second portions 1801 were made of a material including, but not limited to, water-soluble materials as defined in the Pharmaceutical Dosage Form I section. The retaining member second portions 1802 and the retaining member first portions 1801 were connected and together formed a circle, enclosing the unfolding member in a contracted state. The materials of the unfolding member first portion, second portion, and third portion and the materials of the retaining member first portions and second portions were separately fed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted materials of the unfolding member first portion, second portion, and third portion and materials of the retaining member first portions and second portions were fed into a printing head 1, a printing head 2, a printing head 3, a printing head 4, and a printing head 5, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, a G-code set by a three-dimensional slice of a drug model was imported, and the unfolding structure third portion, second portion, and first portion of the pharmaceutical dosage form began to be printed layer by layer, as shown in FIG. 39. Each layer was selectively printed layer by layer by switching between the printing head 1, the printing head 2, and the printing head 3.

After the printing of the unfolding member was completed, the retaining member first portions were printed, and the unfolding member and the retaining member first portions were moved to a contracted shape with the distal ends adjacent to each other using a gripper, a mechanical arm, or other mechanical workpiece, as shown in FIG. 38.

Subsequently, by using a 3D printing head, retaining member second portions 1802 were printed between the distal ends of the retaining member first portions 1801 to connect the distal ends of the two.

The printing process of Example 9 may also be performed in the following order:

Retaining member first portions 1801 and second portions 1802 are printed first, and an unfolding member first portion 1803, an unfolding member second portion 1804, and an unfolding member third portion 1805, which are under a constraint force by the retaining member, are printed within a range surrounded by the retaining member, such that the unfolding member is connected to the retaining member first portions 1801. After the printing was completed, the unfolding member was subjected to a heat treatment to increase the elasticity of an elastic portion.

It should be noted that retaining member second portions 1802 in Example 9 may either be printed layer by layer in the vertical direction using a 3D printing head to achieve the connection between the retaining member first portions 1801, or be printed in the vertical direction using a five-axis 3D printing head.

### Example 10

Specifically, a method for printing a pharmaceutical dosage form 1900 using three-dimensional printing is described in detail with reference to the structure of the pharmaceutical dosage form 1900 shown in FIGs. 40 to 43.

First, a pharmaceutical dosage form as shown in FIG. 34 was printed, comprising a retaining member 1901, an unfolding member first portion 1902, and an unfolding member second portion 1903.

The unfolding member second portion 1903 was made of povidone Kollidon SR, and was in an S-shaped continuously folded shape. The unfolding member first portion 1902 was in the shape of a dumbbell, having a compartment therein in which a drug was loaded. The unfolding member first portion 1902 was made of a material including ethylcellulose (EC), sorbitol (DBS), and titanium dioxide (TiO₂). The retaining member 1901 encased the unfolding member in a contracted state. The retaining member 1901 was made of a material including polyvinylpyrrolidone Kollidon VA64, hydroxypropyl cellulose (HPC), and triethyl citrate (TEC). The material of the unfolding member first portion, the material of the unfolding member second portion, and the material of the retaining member were separately fed into a twin-screw extruder, and the extrusion temperature and screw rotation speed for different functional block functions of heating ends from a discharge port to a feed port were separately set. After mixing, hot melting, and extrusion, the materials were separately conveyed into printing head cylinders of a drug 3D printer, and the melted material of the unfolding member first portion, material of the unfolding member second portion, and material of the retaining member were fed into a printing head 1, a printing head 2, and a printing head 3, respectively.

The temperatures of the cylinder and nozzle of the drug 3D printer were set, with the temperature of the cylinder being 50 to 80 °C and the temperature of the nozzle being 80 to 120 °C. The printing pressure of the printing head was set to 1 to 2 Mpa, and the rotation speed of the conveying screw was set to 0.5 to 2 rps. The D³ software was executed, a G-code set by a three-dimensional slice of a drug model was imported, and the unfolding structure second portion 1903 and the unfolding structure first portion 1902 of the pharmaceutical dosage form began to be printed layer by layer, as shown in FIG. 41.

After the printing of the unfolding member was completed, the unfolding member second portion 1903 was moved to a contracted shape with the distal ends adjacent to each other using a gripper, a mechanical arm, or other mechanical workpieces, as shown in FIG. 40.

Subsequently, by using a 3D printing head, a retaining member was directly printed on the unfolding member in the contracted state, filling the gap in the unfolding member and encasing the unfolding member in the contracted state.

The printing process of Example 10 may also be performed in the following order:

An outer periphery of a retaining member 1901 is printed first, and an unfolding member first portion 1902 and an unfolding member second portion 1903, which are under a constraint force by the retaining member, are printed within a range surrounded by the outer periphery of the retaining member, such that the unfolding member is connected to the outer periphery of the retaining member. After the printing of the unfolding member is completed, a retaining member is directly printed on the unfolding member in the contracted state, filling the gap in the unfolding member and encasing the unfolding member in the contracted state.

### Example 11

The pharmaceutical dosage form of Example 10 was verified for retention effect under simulated gastric acid conditions of a human body, i.e., in 900 mL of a phosphate buffer solution (pH 1.0) at 37 °C.

**Table 2. Size of pharmaceutical dosage form under simulated gastric acid conditions of human body**

| **Original length (mm)** | **Compressed length (mm)** | **Length after opening for 1 h (mm)** |
|---|---|---|
| 13.01 | 9.19 | 16.19 |

As shown in FIGs. 43-44 and Table 2, the pharmaceutical dosage form of Example 10 was gradually unfolded in the phosphate buffer solution to reach a size greater than 16 mm, and was able to maintain it for at least 10 hours, demonstrating a good gastric retention effect.

The pharmaceutical dosage form disclosed herein can be printed on a commercial scale. For example, in some embodiments, the 3D printing method described in the present disclosure can print about 10,000 to about 100,000 oral tablets per hour. In some embodiments, a commercial batch of an oral pharmaceutical dosage form has or substantially conforms to one or more of the predetermined dosage form characteristics described in the present disclosure, including printing uniformity, precision of layer thickness, precision of layer surface area, precision of mass fraction of active ingredient per layer, precision of shape, size and weight of dosage form, precision of content of active ingredient, and precision of release profile of active ingredient. In some embodiments, at least about 80%, such as at least about any one of 85%, 90%, or 95%, of a commercial batch of an oral pharmaceutical dosage form has or substantially conforms to one or more of the predetermined dosage form characteristics described in the present disclosure, including printing uniformity, precision of layer thickness, precision of layer surface area, precision of mass fraction of active ingredient per layer, precision of shape, size and weight of dosage form, precision of content of active ingredient, and precision of release profile of active ingredient.

The pharmaceutical dosage form disclosed in the present application avoids the use of other loading containers such as capsules, reduces the cost, and improves the production efficiency because the retaining member is directly printed to achieve a constraint on the pharmaceutical dosage form structure in a contracted shape. In some embodiments, after the printing of the retaining member of the pharmaceutical dosage form is completed, the outer shell of the pharmaceutical dosage form is encased by inkjet printing, making the outer surface smooth and aesthetically pleasing. Alternatively, LOGOs, such as a company name or abbreviation, a pharmaceutical trade name, a pharmaceutical chemical name or abbreviation, a strength, or a combination thereof, may also be printed on the outer surface of the pharmaceutical dosage form by inkjet printing.

It should be noted that although different portions of the pharmaceutical dosage form and sub-portions of these different portions are mentioned in the above detailed description, this division is only exemplary and not mandatory.

Although the principles of the present invention have been described with reference to the embodiments, it should be understood that these descriptions are made by way of example only and are not intended to limit the scope of the present invention. The contents of the present disclosure are only for purposes of example and illustration, and are not intended to be exhaustive or to limit the scope of the present disclosure in a particular form. Various modifications and variations will be apparent to those skilled in the art. The present disclosure was chosen and described in order to best explain the principles and practical applications of the embodiments, thereby enabling those skilled in the art to understand the various embodiments and various modifications made for a particular application. The scope of the present disclosure is defined by the following claims or equivalents thereof.

## Claims

1. A pharmaceutical dosage form, comprising:
a pharmaceutical unit comprising a drug;
at least one unfolding member connected to the pharmaceutical unit and configured to have a contracted shape proximal to the pharmaceutical unit and an unfolding shape distal to the pharmaceutical unit; and
a retaining member connected to the at least one unfolding member and applying a constraint force to the at least one unfolding member to enable the unfolding member to be in the contracted shape;
wherein the retaining member comprises a water-soluble material, and the retaining member is configured to remove the constraint force from the unfolding member when the water-soluble material is dissolved, such that the at least one unfolding member can change from the contracted shape to the unfolding shape.

2. The pharmaceutical dosage form according to claim 1, wherein the unfolding member comprises at least one elastic portion configured such that the unfolding member is in the contracted shape proximal to the pharmaceutical unit under the action of the constraint force, and returns to the unfolding shape distal to the pharmaceutical unit when the constraint force is withdrawn.

3. The pharmaceutical dosage form according to claim 1, wherein the retaining member at least partially encases the pharmaceutical unit and the at least one unfolding member, such that the at least one unfolding member is in the contracted shape.

4. The pharmaceutical dosage form according to claim 1, wherein the retaining member is disposed between the pharmaceutical unit and the unfolding member and is connected to the pharmaceutical unit, such that the unfolding member is in the contracted shape.

5. The pharmaceutical dosage form according to claim 1, comprising a plurality of unfolding members, wherein the retaining member is connected to at least two unfolding members, such that the at least two unfolding members are in the contracted shape.

6. The pharmaceutical dosage form according to claim 2, wherein the elastic portion is located at one end of the unfolding member adjacent to the pharmaceutical unit.

7. The pharmaceutical dosage form according to claim 1, wherein the unfolding member has a portion with reduced thickness at the end adjacent to the pharmaceutical unit.

8. The pharmaceutical dosage form according to claim 1, wherein the unfolding member has a strip, petal, or rod shape.

9. The pharmaceutical dosage form according to claim 1, comprising a plurality of unfolding members substantially uniformly distributed along the periphery of the pharmaceutical unit.

10. The pharmaceutical dosage form according to claim 1, wherein the pharmaceutical unit comprises a hollow compartment.

11. The pharmaceutical dosage form according to claim 1, wherein the pharmaceutical unit comprises an outer shell and a drug-containing inner core, wherein the outer shell comprises the same host material as the unfolding member.

12. A method for preparing a pharmaceutical dosage form using a three-dimensional printing process, comprising:
printing a pharmaceutical unit and an unfolding member layer by layer, such that the unfolding member is connected to the pharmaceutical unit;
configuring the unfolding member to have a contracted shape proximal to the pharmaceutical unit from an unfolding shape; and
directly printing a retaining member, such that the retaining member is connected to at least one unfolding member and applies a constraint force to the at least one unfolding member to enable the unfolding member to be in the contracted shape;
wherein the retaining member comprises a water-soluble material, and the retaining member is configured to remove the constraint force from the unfolding member when the water-soluble material is dissolved, such that the at least one unfolding member can change from the contracted shape to the unfolding shape.

13. The method according to claim 12, wherein the at least one unfolding member is printed to comprise at least one elastic portion configured such that the unfolding member is in the contracted shape proximal to the pharmaceutical unit under the action of the constraint force, and returns to the unfolding shape distal to the pharmaceutical unit when the constraint force is withdrawn.

14. The method according to claim 12, wherein the printing the retaining member comprises:
printing the retaining member, such that the retaining member at least partially encases or bonds with the pharmaceutical unit and the at least one unfolding member, such that the at least one unfolding member is in the contracted shape.

15. The method according to claim 12, wherein the printing the retaining member comprises:
printing the retaining member, such that the retaining member is disposed between the pharmaceutical unit and the unfolding member and is connected to the pharmaceutical unit, such that the unfolding member is in the contracted shape.

16. The method according to claim 12, wherein the steps of printing the at least one unfolding member and printing the retaining member are performed simultaneously or alternately by different print heads.

17. The method according to claim 12, wherein the unfolding member is printed to have a portion with reduced thickness at one end adjacent to the pharmaceutical unit.

18. The method according to claim 12, wherein the printing the at least one unfolding member comprises:
printing the at least one unfolding member in the unfolding shape; and
moving the at least one unfolding member, such that the unfolding member is in the contracted shape.

19. The method according to claim 12, wherein the pharmaceutical unit comprises a first portion comprising a drug and a second portion comprising no drug, wherein the first portion comprising the drug is printed after the step of printing the retaining member is completed.

20. A pharmaceutical dosage form, comprising:
a pharmaceutical unit comprising an outer shell layer and a drug layer, wherein at least a portion of the pharmaceutical unit has a bent or folded shape; and
a retaining member connected to the pharmaceutical unit and applying a constraint force to the pharmaceutical unit to enable at least a portion of the pharmaceutical unit to retain the bent or folded shape;
wherein the retaining member comprises a water-soluble material, and the retaining member is configured to remove the constraint force from the pharmaceutical unit when the water-soluble material is dissolved, thereby allowing at least a portion of the pharmaceutical unit to unfold from the bent or folded shape.

21. The pharmaceutical dosage form according to claim 20, wherein the pharmaceutical unit is configured to have a helical shape with a radius of curvature gradually increasing from the inside to the outside, and the retaining member at least partially fills a gap of the helical shape of the pharmaceutical unit.

22. The pharmaceutical dosage form according to claim 20, wherein the pharmaceutical unit comprises the outer shell layer and the drug layer, wherein the outer shell layer is composed of a hydrophobic material.

23. The pharmaceutical dosage form according to claim 22, wherein the drug layer comprises a hydrophilic material.

24. The pharmaceutical dosage form according to claim 23, wherein the drug layer comprises a first portion and a second portion, wherein the first portion and the second portion comprise different drugs.

25. The pharmaceutical dosage form according to claim 20, wherein the pharmaceutical unit is generally in an elongated shape when unfolded.

26. A method for preparing a pharmaceutical dosage form using a three-dimensional printing process, comprising:
printing a pharmaceutical unit comprising a drug, such that at least a portion of the pharmaceutical unit has a bent or folded shape; and
printing a retaining member, such that the retaining member is connected to the pharmaceutical unit and applies a constraint force to the pharmaceutical unit to enable at least a portion of the pharmaceutical unit to retain the bent or folded shape;
wherein the retaining member comprises a water-soluble material, and the retaining member is configured to remove the constraint force from the pharmaceutical unit when the water-soluble material is dissolved, thereby allowing at least a portion of the pharmaceutical unit to unfold from the bent or folded shape.

27. The method according to claim 26, wherein the pharmaceutical unit is configured to have a helical shape with a radius of curvature gradually increasing from the inside to the outside, and the retaining member at least partially fills a gap of the helical shape of the pharmaceutical unit.

28. The method according to claim 26, wherein an outer shell layer is composed of a hydrophobic material.

29. A pharmaceutical dosage form, comprising:
an unfolding member comprising at least one elastic portion configured such that the unfolding member is in a contracted shape under the action of a constraint force, and returns to an unfolding shape when the constraint force is withdrawn; and
a retaining member surrounded the unfolding member and applying the constraint force to the unfolding member to enable the unfolding member to retain the contracted shape;
wherein the retaining member comprises a water-soluble material, and the retaining member is configured to remove the constraint force from the unfolding member when the water-soluble material is dissolved, thereby allowing the unfolding member to at least partially unfold.

30. The pharmaceutical dosage form according to claim 29, wherein the retaining member comprises a first portion and a second portion, the unfolding member is connected to the first portion, the second portion comprises a water-soluble material, and the retaining member removes the constraint force from the unfolding member when the water-soluble material of the second portion is dissolved, thereby allowing the unfolding member to at least partially unfold.

31. The pharmaceutical dosage form according to claim 30, wherein the first portion comprises a first drug and the second portion comprises a second drug.

32. The pharmaceutical dosage form according to claim 31, wherein the first portion comprises at least one compartment, and the first drug is disposed in the compartment.

33. The pharmaceutical dosage form according to claim 30, wherein the unfolding member comprises a first portion and a second portion, and the first portion of the unfolding member has a dissolution rate in gastric juice less than the second portion of the retaining member in gastric juice.

34. The pharmaceutical dosage form according to claim 33, wherein the first portion of the unfolding member comprises a drug.

35. The pharmaceutical dosage form according to claim 33, wherein the unfolding member further comprises a third portion, wherein the first portion of the unfolding member is connected to the second portion of the unfolding member by the third portion of the unfolding member, and the first portion of the unfolding member is disconnected from the second portion when the third portion is dissolved.

36. The pharmaceutical dosage form according to claim 35, wherein the second portion and the third portion of the unfolding member comprise a hydrophobic material and an enteric material.

37. The pharmaceutical dosage form according to claim 33, wherein the first portion of the unfolding member substantially extends along a first direction, the second portion of the unfolding member substantially extends along a second direction, and the first portion and the second portion of the unfolding member are cross-connected to each other.

38. The pharmaceutical dosage form according to claim 37, wherein the first portion of the unfolding member comprises at least one internal compartment, wherein a drug is disposed in the internal compartment.

39. The pharmaceutical dosage form according to claim 29, wherein the elastic portion of the unfolding member is configured to substantially extend along a plane when the unfolding member is in a contracted configuration.

40. The pharmaceutical dosage form according to claim 29, wherein the pharmaceutical dosage form has a length greater than or equal to 16 mm at least along one direction when the water-soluble material of the retaining member is at least partially dissolved.

41. The pharmaceutical dosage form according to claim 29, wherein the unfolding member is configured to fully unfold within at least 10 min after entering gastric juice.

42. A method for preparing a pharmaceutical dosage form using a three-dimensional printing process, comprising:
printing an unfolding member comprising at least one elastic portion configured such that the unfolding member is in a contracted shape under the action of a constraint force, and returns to an unfolding shape when the constraint force is withdrawn;
applying the constraint force to the unfolding member to enable the unfolding member to retain the contracted shape; and
printing a retaining member to retain the constraint force on the unfolding member by the retaining member to enable the unfolding member to retain the contracted shape;
wherein the retaining member comprises a water-soluble material, and the retaining member is configured to remove the constraint force from the unfolding member when the water-soluble material is dissolved, thereby allowing the unfolding member to at least partially unfold.

43. The method according to claim 42, wherein the retaining member comprises a first portion and a second portion, and the method further comprises:
printing a first portion of the retaining member after printing the unfolding member, such that the unfolding member is connected to the first portion of the retaining member; and
applying the constraint force to the unfolding member by the first portion of the retaining member to enable the unfolding member to retain the contracted shape.

44. The method according to claim 43, further comprising:
printing a second portion of the retaining member, wherein the second portion comprises a water-soluble material, and the retaining member removes the constraint force from the unfolding member when the water-soluble material of the second portion is dissolved, thereby allowing the unfolding member to at least partially unfold.

45. A method for preparing a pharmaceutical dosage form using a three-dimensional printing process, comprising:
printing a retaining member; and
printing an unfolding member under the action of a constraint force of the retaining member within a range surrounded by the retaining member, such that the unfolding member is connected to the retaining member, wherein the unfolding member comprises at least one elastic portion configured to enable the unfolding member to at least partially unfold when the constraint force applied by the retaining member is removed;
wherein the retaining member comprises a water-soluble material, and the retaining member is configured to remove the constraint force from the unfolding member when the water-soluble material is dissolved, thereby allowing the unfolding member to at least partially unfold.
